# EUROPEAN PATENT APPLICATION

(11) **EP 2 128 249 A1**
(43) Date of publication of application: **02.12.2009**
(21) Application number: 08711706.5
(22) Date of filing: 14.02.2008
(51) Int. Cl.: C12N 15/00, C07K 16/18, C07K 19/00, C12N 15/09, C12P 21/08, G01N 33/543, G01N 33/566

(54) **ZINC OXIDE-BINDING ANTIBODY AND USE THEREOF**

(30) Priority: 15.02.2007 JP 2007035073
(71) Applicant: Tohoku University, Aoba-ku Sendai-shi Miyagi 980-8577 (JP)
(72) Inventor: UMETSU, Mitsuo, Sendai-shi Miyagi 980-8577 (JP); KUMAGAI, Izumi, Sendai-shi Miyagi 980-8577 (JP); ASANO, Ryutaro, Sendai-shi Miyagi 980-8577 (JP); NAKANISHI, Takeshi, Sendai-shi Miyagi 980-8577 (JP)
(74) Representative: Denison, Christopher Marcus
(86) International application number: PCT/JP2008/052917
(87) International publication number: WO 2008/099968

(57) **Abstract**

The present invention relates to a zinc oxide-binding antibody having high stability and binding activity, and high-throughput sensing technology using the antibody, such as biosensors. Specifically, the present invention relates to a peptide-grafted antibody that contains a zinc oxide-recognizing peptide in the CDR H-1 region of a camel antibody, and a solid support (e.g., a biosensor and a protein chip) containing a zinc oxide layer on which the antibody has been immobilized.

SEQUENCE LISTING

<110>  Tohoku University

<120>  Antibody capable of binding to zinc oxide and uses thereof

<130>  PH-3427-PCT

<150>  JP2007-035073
<151>  2007-02-15

<160>  55

<170>  PatentIn version 3.4

<210>  1
<211>  12
<212>  PRT
<213>  Artificial

<220>
<223> Inventor: Umetsu, Mitsuo; Kumagai, Izumi; Asano, Ryutaro; Nakanishi,
Takeshi

<220>
<223>  ZnO recognition peptide (ZnO1)

<400>  1

Glu Ala His Val Met His Lys Val Ala Pro Arg Pro
1               5                   10


<210>  2
<211>  12
<212>  PRT
<213>  Artificial

<220>
<223>  ZnO recognition peptide (ZnO2)

<400>  2

Gln Asn Thr Ala Thr Ala Val Ser Arg Leu Ser Pro

1                   5                   10


<210>  3
<211>  12
<212>  PRT
<213>  Artificial

<220>
<223>  ZnO recognition peptide (ZnO3)

<400>  3

Ala Thr His Thr Asn Gln Thr His Ala Leu Tyr Arg
1               5                   10


<210>  4
<211>  12
<212>  PRT
<213>  Artificial

<220>
<223>  ZnO recognition peptide (ZnO4)

<400>  4

Val Ser Asn His Lys Ala Leu Asp Tyr Pro Thr Arg
1               5                   10


<210>  5
<211>  12
<212>  PRT
<213>  Artificial

<220>
<223>  ZnO recognition peptide (ZnO5)

<400>  5

Asp Ser Gly Arg Tyr Ser Met Thr Asn His Tyr Ser
1               5                   10

<210> 6
<211> 7
<212> PRT
<213> Artificial

<220>
<223> ZnO recognition sequence

<400> 6

Glu Ala His Val Met His Lys
1               5


<210> 7
<211> 127
<212> PRT
<213> Artificial

<220>
<223> cAb BCII-10

<400> 7

Gln Val Gln Leu Val Glu Ser Gly Gly Gly Ser Val Gln Ala Gly Gly
1               5                   10                  15


Ser Leu Arg Leu Ser Cys Thr Ala Ser Gly Gly Ser Glu Tyr Ser Tyr
            20                  25                  30


Ser Thr Phe Ser Leu Gly Trp Phe Arg Gln Ala Pro Gly Gln Glu Arg
        35                  40                  45


Glu Ala Val Ala Ala Ile Ala Ser Met Gly Gly Leu Thr Tyr Tyr Ala
    50                  55                  60


Asp Ser Val Lys Gly Arg Phe Thr Ile Ser Arg Asp Asn Ala Lys Asn
65                  70                  75                  80

Thr Val Thr Leu Gln Met Asn Asn Leu Lys Pro Glu Asp Thr Ala Ile
                85                  90                  95

Tyr Tyr Cys Ala Ala Val Arg Gly Tyr Phe Met Arg Leu Pro Ser Ser
            100                 105                 110

His Asn Phe Arg Tyr Trp Gly Gln Gly Thr Gln Val Thr Val Ser
        115                 120                 125

<210>  8
<211>  4
<212>  PRT
<213>  Artificial

<220>
<223>  ZnO binding motif

<220>
<221>  misc_feature
<222>  (2)..(3)
<223>  Xaa can be any naturally occurring amino acid

<400>  8

His Xaa Xaa His
1

<210>  9
<211>  10
<212>  PRT
<213>  Artificial

<220>
<223>  H-3 introduced sequence

<220>
<221>  misc_feature

<222>  (2)..(3)
<223>  X=G or L or R or V

<220>
<221>  misc_feature
<222>  (5)..(6)
<223>  X=G or L or R or V

<220>
<221>  misc_feature
<222>  (8)..(9)
<223>  X=G or L or R or V

<400>  9

His Xaa Xaa His Xaa Xaa His Xaa Xaa His
1               5                   10


<210>  10
<211>  10
<212>  PRT
<213>  Artificial

<220>
<223>  H-3 introduced sequence of high affinity mutant (3D2)

<400>  10

His Leu Gly His Gly Gly His Arg Leu His
1               5                   10


<210>  11
<211>  10
<212>  PRT
<213>  Artificial

<220>
<223>  H-3 introduced sequence of high affinity mutant (3E2)

<400>  11

His Leu Gly His Gly Gly His Gly Leu His

1                   5                   10

<210>  12
<211>  10
<212>  PRT
<213>  Artificial

<220>
<223>  H-3 introduced sequence of high affinity mutant (4D4)

<400>  12

His Val Gly His Gly Leu His Gly Val Arg
1               5                   10

<210>  13
<211>  10
<212>  PRT
<213>  Artificial

<220>
<223>  H-3 introduced sequence of high affinity mutant (4F2)

<400>  13

His Leu Gly His Gly Leu His Arg Val His
1               5                   10

<210>  14
<211>  132
<212>  PRT
<213>  Artificial

<220>
<223>  4F2

<400>  14

Gln Val Gln Leu Val Glu Ser Gly Gly Gly Ser Val Gln Ala Gly Gly
1               5                   10                  15

Ser Leu Arg Leu Ser Cys Thr Ala Ser Gly Gly Ser Glu Ala His Val
            20                  25                  30

Met His Lys Val Ala Pro Arg Pro Ser Leu Gly Trp Phe Arg Gln Ala
        35                  40                  45

Pro Gly Gln Glu Arg Glu Ala Val Ala Ala Ile Ala Ser Met Gly Gly
    50                  55                  60

Leu Thr Tyr Tyr Ala Asp Ser Val Lys Gly Arg Phe Thr Ile Ser Arg
65                  70                  75                  80

Asp Asn Ala Lys Asn Thr Val Thr Leu Gln Met Asn Asn Leu Lys Pro
                85                  90                  95

Glu Asp Thr Ala Ile Tyr Tyr Cys Ala Ala Val Arg Gly His Leu Gly
            100                 105                 110

His Gly Leu His Arg Val His Phe Arg Tyr Trp Gly Gln Gly Thr Gln
        115                 120                 125

Val Thr Val Ser
    130

<210>  15
<211>  132
<212>  PRT
<213>  Artificial

<220>
<223>  VHH1

<400>  15

Gln Val Gln Leu Val Glu Ser Gly Gly Gly Ser Val Gln Ala Gly Gly

1               5                   10                  15

Ser Leu Arg Leu Ser Cys Thr Ala Ser Gly Gly Ser Glu Ala His Val
            20                  25                  30

Met His Lys Val Ala Pro Arg Pro Ser Leu Gly Trp Phe Arg Gln Ala
        35                  40                  45

Pro Gly Gln Glu Arg Glu Ala Val Ala Ala Ile Ala Ser Met Gly Gly
    50                  55                  60

Leu Thr Tyr Tyr Ala Asp Ser Val Lys Gly Arg Phe Thr Ile Ser Arg
65                  70                  75                  80

Asp Asn Ala Lys Asn Thr Val Thr Leu Gln Met Asn Asn Leu Lys Pro
                85                  90                  95

Glu Asp Thr Ala Ile Tyr Tyr Cys Ala Ala Val Arg Gly Tyr Phe Met
            100                 105                 110

Arg Leu Pro Ser Ser His Asn Phe Arg Tyr Trp Gly Gln Gly Thr Gln
        115                 120                 125

Val Thr Val Ser
    130

<210>  16
<211>  130
<212>  PRT
<213>  Artificial

<220>
<223>  VHH2

<400>  16

Gln Val Gln Leu Val Glu Ser Gly Gly Gly Ser Val Gln Ala Gly Gly
1               5                   10                  15

Ser Leu Arg Leu Ser Cys Thr Ala Ser Gly Gly Ser Glu Tyr Ser Tyr
            20                  25                  30

Ser Thr Phe Ser Leu Gly Trp Phe Arg Gln Ala Pro Gly Gln Glu Arg
        35                  40                  45

Glu Ala Val Ala Ala Ile Ala Glu Ala His Val Met His Lys Val Ala
    50                  55                  60

Pro Arg Pro Asp Ser Val Lys Gly Arg Phe Thr Ile Ser Arg Asp Asn
65                  70                  75                  80

Ala Lys Asn Thr Val Thr Leu Gln Met Asn Asn Leu Lys Pro Glu Asp
                85                  90                  95

Thr Ala Ile Tyr Tyr Cys Ala Ala Val Arg Gly Tyr Phe Met Arg Leu
            100                 105                 110

Pro Ser Ser His Asn Phe Arg Tyr Trp Gly Gln Gly Thr Gln Val Thr
        115                 120                 125

Val Ser
    130

<210>  17
<211>  127
<212>  PRT
<213>  Artificial

<220>
<223>  VHH3

<400> 17

Gln Val Gln Leu Val Glu Ser Gly Gly Gly Ser Val Gln Ala Gly Gly
1               5                   10                  15

Ser Leu Arg Leu Ser Cys Thr Ala Ser Gly Gly Ser Glu Tyr Ser Tyr
            20                  25                  30

Ser Thr Phe Ser Leu Gly Trp Phe Arg Gln Ala Pro Gly Gln Glu Arg
        35                  40                  45

Glu Ala Val Ala Ala Ile Ala Ser Met Gly Gly Leu Thr Tyr Tyr Ala
    50                  55                  60

Asp Ser Val Lys Gly Arg Phe Thr Ile Ser Arg Asp Asn Ala Lys Asn
65                  70                  75                  80

Thr Val Thr Leu Gln Met Asn Asn Leu Lys Pro Glu Asp Thr Ala Ile
                85                  90                  95

Tyr Tyr Cys Ala Ala Val Glu Ala His Val Met His Lys Val Ala Pro
            100                 105                 110

Arg Pro Phe Arg Tyr Trp Gly Gln Gly Thr Gln Val Thr Val Ser
        115                 120                 125

<210>  18
<211>  16
<212>  PRT
<213>  Artificial

<220>
<223>  cAb BCII-10 CDR H-3 region (positions 102-117), Fig.9

<400>  18

Val Arg Gly Tyr Phe Met Arg Leu Pro Ser Ser His Asn Phe Arg Tyr
1               5                   10                  15

<210>  19
<211>  16
<212>  PRT
<213>  Artificial

<220>
<223>  cAb BCII-10 CDR H-3 region introduced sequence, Fig.9

<220>
<221>  misc_feature
<222>  (5)..(6)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (11)..(12)
<223>  Xaa can be any naturally occurring amino acid

<400>  19

Val Arg Gly His Xaa Xaa His Xaa Xaa His Xaa Xaa His Phe Arg Tyr
1               5                   10                  15

<210>  20
<211>  12
<212>  PRT
<213>  Artificial

<220>
<223>  Au recognition peptide

<400>  20

Leu Lys Ala His Leu Pro Pro Ser Arg Leu Pro Ser
1               5                   10

<210>  21
<211>  31
<212>  DNA
<213>  Artificial

<220>
<223>  primer

<220>
<221>  misc_feature
<222>  (1)..(3)
<223>  n is a, c, g, or t

<400>  21
nnnccatggc ccaggttcag ctggttgaaa g                                    31

<210>  22
<211>  96
<212>  DNA
<213>  Artificial

<220>
<223>  primer

<400>  22
caggttcagc tggttgaaag cggcggcggc agcgtgcagg cgggcggcag cctgcgcctg      60

agctgcaccg cgagcggcgg cagcgaatat agctat                                96

<210>  23
<211>  87
<212>  DNA
<213>  Artificial

<220>
<223>  primer

<400> 23
aattgctgcc actgcttcgc gttcctggcc cggcgcctgg cgaaaccagc ccaggctaaa 60

ggtgctatag ctatattcgc tgccgcc 87


<210> 24
<211> 98
<212> DNA
<213> Artificial

<220>
<223> primer

<400> 24
gcgaagcagt ggcagcaatt gcgagcatgg gcggcctgac ctattatgcg gatagcgtga 60

aaggccgctt taccattagc cgcgataacg cgaaaaac 98


<210> 25
<211> 91
<212> DNA
<213> Artificial

<220>
<223> primer

<400> 25
gcttccactg ctgcgcaata ataaatcgcg gtatcttccg gtttcaggtt gttcatctgc 60

agggtcacgg tgtttttcgc gttatcgcgg c 91


<210> 26
<211> 93
<212> DNA
<213> Artificial

<220>
<223> primer

<400> 26

tattgcgcag cagtggaagc gcatgtgatg cataaagtgg cgccgcgccc gtttcgctat 60

tggggccagg gcacccaggt gaccgtgagc agc 93

<210> 27
<211> 27
<212> DNA
<213> Artificial

<220>
<223> primer

<220>
<221> misc_feature
<222> (1)..(3)
<223> n is a, c, g, or t

<400> 27
nnnccgcgga tgaaacggta acctggg 27

<210> 28
<211> 50
<212> DNA
<213> Artificial

<220>
<223> primer

<400> 28
gctcggcagg cgcataaaat agccgcgcac tgctgcgcaa taataaatcg 50

<210> 29
<211> 50
<212> DNA
<213> Artificial

<220>
<223> primer

<400> 29

tattttatgc gcctgccgag cagccataac tttcgctatt ggggccaggg 50

<210> 30
<211> 55
<212> DNA
<213> Artificial

<220>
<223> primer

<400> 30
gcgccacttt atgcatcaca tgcgcttcgc taccaccgct cgcggtgcag ctcag 55

<210> 31
<211> 49
<212> DNA
<213> Artificial

<220>
<223> primer

<400> 31
tgtgatgcat aaagtggcgc cgcgtccgag cctgggctgg tttcgccag 49

<210> 32
<211> 49
<212> DNA
<213> Artificial

<220>
<223> primer

<400> 32
gcgccacttt atgcatcaca tgcgcttccg caattgctgc cactgcttc 49

<210> 33
<211> 49
<212> DNA
<213> Artificial

<220>
<223>  primer

<400>  33
tgtgatgcat aaagtggcgc cgcgtccgga tagcgtgaaa ggccgcttt                49


<210>  34
<211>  67
<212>  DNA
<213>  Artificial

<220>
<223>  primer


<220>
<221>  misc_feature
<222>  (1)..(3)
<223>  n is a, c, g, or t

<400>  34
nnnccatggc cgaagcgcat gtgatgcata aagtggcgcc gcgtccgcag gttcagctgg      60

ttgaaag                                                                67


<210>  35
<211>  31
<212>  DNA
<213>  Artificial

<220>
<223>  primer


<220>
<221>  misc_feature
<222>  (1)..(3)
<223>  n is a, c, g, or t

<400>  35
nnnccatggc ccaggttcag ctggttgaaa g                                     31

<210> 36
<211> 70
<212> DNA
<213> Artificial

<220>
<223> primer

<400> 36
attgcgcagc agtgcgcggt crcsktsktc rcsktsktcr csktsktcrc tttcgctatt 60

ggggccaggg 70

<210> 37
<211> 70
<212> DNA
<213> Artificial

<220>
<223> primer

<400> 37
ccctggcccc aatagcgaaa gygamsamsg ygamsamsgy gamsamsgyg accgcgcact 60

gctgcgcaat 70

<210> 38
<211> 27
<212> DNA
<213> Artificial

<220>
<223> primer

<220>
<221> misc_feature
<222> (1)..(3)
<223> n is a, c, g, or t

<400> 38

nnnccgcgga tgaaacggta acctggg                                    27

<210>  39
<211>  29
<212>  PRT
<213>  Artificial

<220>
<223>  linker sequence (Llama IgG2 upper hinge)

<400>  39

Glu Pro Lys Ile Pro Gln Pro Gln Pro Lys Pro Gln Pro Gln Pro Gln
1               5                   10                  15

Pro Gln Pro Gln Pro Lys Pro Gln Pro Lys Pro Glu Pro
            20                  25

<210>  40
<211>  25
<212>  DNA
<213>  Artificial

<220>
<223>  primer

<400>  40
gaaattaata cgactcacta taggg                                      25

<210>  41
<211>  79
<212>  DNA
<213>  Artificial

<220>
<223>  primer

<400>  41
ttgcggttgt ggttgcggtt gcggtttcgg ctgcggctgc ggaattttcg gttcggccga      60

tgaaacggta acctgggtg                                                    79

<210>  42
<211>  81
<212>  DNA
<213>  Artificial

<220>
<223>  primer

<400>  42
caaccgcaac cacaaccgca accgcagccg aaaccgcagc cgaaaccgga accgggatcc      60

caggttcagc tggttgaaag c                                                81

<210>  43
<211>  19
<212>  DNA
<213>  Artificial

<220>
<223>  primer

<400>  43
gctagttatt gctcagcgg                                                   19

<210>  44
<211>  26
<212>  DNA
<213>  Artificial

<220>
<223>  primer

<220>
<221>  misc_feature
<222>  (1)..(3)
<223>  n is a, c, g, or t

<400>  44

nnnccatggc cgatatccag ctgcag 26

<210> 45
<211> 27
<212> DNA
<213> Artificial

<220>
<223> primer

<220>
<221> misc_feature
<222> (1)..(3)
<223> n is a, c, g, or t

<400> 45
nnnccgcgga gacggtgacg agggtgc 27

<210> 46
<211> 48
<212> DNA
<213> Artificial

<220>
<223> primer

<400> 46
gcgccacttt atgcatcaca tgcgcttcgc ccatgtactc gaggcggt 48

<210> 47
<211> 48
<212> DNA
<213> Artificial

<220>
<223> primer

<400> 47
tgtgatgcat aaagtggcgc cgcgtccgcg catctcgatc acccgcga 48

<210> 48
<211> 48
<212> DNA
<213> Artificial

<220>
<223> primer

<400> 48
gcgccacttt atgcatcaca tgcgcttcgt tcgcgcagta gtaggtgg 48


<210> 49
<211> 48
<212> DNA
<213> Artificial

<220>
<223> primer

<400> 49
tgtgatgcat aaagtggcgc cgcgtccgtg gggccagggc accctcgt 48


<210> 50
<211> 27
<212> DNA
<213> Artificial

<220>
<223> primer


<220>
<221> misc_feature
<222> (1)..(3)
<223> n is a, c, g, or t

<400> 50
nnnccatggc cgatatcgtc ctgaccc 27


<210> 51

<211>  27
<212>  DNA
<213>  Artificial

<220>
<223>  primer


<220>
<221>  misc_feature
<222>  (1)..(3)
<223>  n is a, c, g, or t

<400>  51
nnnccgcggc cttgatctcc agcttgg                                          27


<210>  52
<211>  47
<212>  DNA
<213>  Artificial

<220>
<223>  primer

<400>  52
gcgccacttt atgcatcaca tgcgcttcct tgatcaggag gcgcggg                    47


<210>  53
<211>  48
<212>  DNA
<213>  Artificial

<220>
<223>  primer

<400>  53
tgtgatgcat aaagtggcgc cgcgtccggg gattccgtcg cgcttcag                   48


<210>  54
<211>  49
<212>  DNA

<213>  Artificial

<220>
<223>  primer

<400>  54
gcgccacttt atgcatcaca tgcgcttcgc agaagtacat gccgaagtc                49


<210>  55
<211>  47
<212>  DNA
<213>  Artificial

<220>
<223>  primer

<400>  55
tgtgatgcat aaagtggcgc cgcgtccgtt cggcggcggc accaagc                47

## Description

### Technical Field

The present invention relates to a zinc oxide-binding camel antibody capable of specifically binding to zinc oxide, an expression vector for the antibody, and a solid support (e.g., biosensors and protein chips) containing a zinc oxide layer to which the antibody has been immobilized.

### Background Art

Biomolecules are involved in molecular recognition mechanisms with extremely high specificity, such as a mechanism for enzyme-substrate recognition and a mechanism for antigen-antibody recognition. Biomolecules exert such functions with high safety and high accuracy under extremely mild conditions. Thus, the use of such functions has been expected in various fields including medicine, food, the environment, and the like.

A key for material development using such recognition mechanisms of biomolecules is to stably immobilize biomolecules such as proteins on inorganic materials. To date, protein immobilization methods using coupling agents are known concerning coated gold surfaces. However, the methods result in poor immobilization reaction rates leading to lowered protein activity. In contrast, the present inventors have screened a human peripheral blood cell Fab gene library to obtain a gold-binding protein (gold-binding VL or VH), and thus succeeded in immobilization of the protein on gold substrates (JP Patent Publication No. 2005-314411 A and JP Patent Publication No. 2005-312446 A).

Metal oxides are important materials, and they are used for many optical-electronic devices. Particularly, zinc oxide is transparent and can be used as an insulator or a semiconductor. So, it can be a material that can be used for various applications such as biosensing. If biomolecules (proteins) could be immobilized on zinc oxide conveniently and directly, the products would be extremely useful for development of high-throughput sensing technology. The present inventors have obtained peptides having high and specific binding activity to zinc oxide by screening phage display libraries (Umetsu et al., Advanced Materials, 17, pp.2571-2575, (2005)). Thus, the present inventors have succeeded in the patterning of zinc oxide particles using the peptides (JP Patent Publication No. 2006-225294 A).

Furthermore, the present inventors have attempted to develop a material-specific antibody by fusing the above-mentioned material-recognizing peptide (e.g., a gold-binding protein and a zinc oxide-binding peptide) to an anti-hen egg white lysozyme (HEL) antibody using a CDR-grafting technique which is employed for antibody humanization technique (The 3^{rd} Tohoku University Bioscience Symposium (2006) Lecture Summaries, p. 240 PS-189, "Research Concerning Multi-functional Antibody Targeting Engineering Material"; and The 3rd Tohoku University Bioscience Symposium (2006) Lecture Summaries, p. 241 PS-190, "Creation of Inorganic Material-binding Antibody by Grafting of Functional Peptide"). However, a zinc oxide-binding antibody fused to an HEL antibody is unstable and inappropriate for practical uses such as in biosensors or protein chips.

A camel antibody is thought to be inappropriate for CDR grafting of a peptide, since it lacks canonical structure and has a disulfide bond between the complementarity determining regions (CDRs). Recently, Saerens et al., have developed a camel antibody suitable for CDR grafting (Saerens et al., J. Mol. Biol., 352, pp. 597-607, (2005)), and the use of such camel antibody in medical fields, such as for targeting cancer cells, is becoming expected. However, the use of such camel antibody for development of materials for biosensors, for example, has never been reported.

### Summary of the Invention

### Problems to be Solved by the Invention

An object of the present invention is to provide a zinc oxide-binding antibody having high stability and binding activity and use it by stably binding the antibody to zinc oxide in order to develop high-throughput sensing technology such as immunobiosensors.

### Means for Solving the Problems

As a result of intensive studies to solve the above object, the present inventors have discovered that a stable zinc oxide-binding antibody can be prepared using a camel antibody. Moreover, the present inventors have further enhanced the affinity of the antibody using *in vitro* affinity maturation and thus succeeded in preparation of a zinc oxide-binding antibody having high stability and binding activity, which is appropriate for practical uses.

That is, the present invention relates to a zinc oxide-binding antibody, comprising:
a peptide-grafted antibody that contains a zinc oxide-recognizing peptide having an amino acid sequence comprising EAHVMHK in the CDR H-1 region of a camel antibody; or
a mutant that has another mutation in the CDR H-3 region of the above peptide-grafted antibody and has an affinity for zinc oxide higher than that of the above peptide-grafted antibody.

The above mutant includes, for example, a mutant that contains a peptide comprising HXXHXXHXXH or HXXHXXHXXR (wherein X is G, L, R, or V) in the CDR H-3 region and a mutant that contains a peptide comprising HLGHGGHRLH, HLGHGGHGLH, HVGHGLHGVR, or HLGHGLHRVH in the CDR H-3 region.

The zinc oxide-binding antibody of the present invention has a dissociation equilibrium constant K_{d} for zinc oxide preferably lower than 1.7 × 10⁻⁷ [M], and more preferably lower than 9.5 × 10⁻⁹ [M].

The above zinc oxide-recognizing peptide may be, preferably a peptide comprising 7 to 20 amino acids containing EAHVMHK, and particularly a peptide comprising approximately 7 to 12 amino acids, such as EAHVMHKVAPRP

In one embodiment, the zinc oxide-binding antibody of the present invention is an antibody that contains a zinc oxide-recognizing peptide comprising EAHVMHKVAPRP in the CDR H-1 region of a camel antibody and a peptide comprising HLGHGLHRVH in the CDR H-3 region.

In addition, the above camel antibody preferably has no disulfide bond between CDRs.

The present invention also provides an expression vector comprising a gene encoding the above zinc oxide-binding antibody. An example of such vector is an expression vector comprising a gene encoding the amino acid sequence shown in SEQ ID NO: 14.

The present invention also provides a method for producing a zinc oxide-binding antibody, comprising culturing host cells into which the above expression vector has been introduced and then collecting the zinc oxide-binding antibody from the culture.

Furthermore, the present invention provides a solid support comprising a zinc oxide layer on which the above zinc oxide-binding antibody has been immobilized (e.g., particles or substrates such as protein chips).

Furthermore, the present invention also provides a biosensor having a solid support comprising a zinc oxide layer on which the above zinc oxide-binding antibody has been immobilized and a means for detecting intermolecular interactions via the above antibody.

Furthermore, the present invention also provides a protein comprising the above zinc oxide-binding antibody which is fused to a second protein having a material-recognizing sequence. The term "second protein having a material-recognizing sequence" refers to a protein containing a sequence that specifically recognizes a material (e.g., metal or a metal oxide) so as to specifically bind to the material, as in the case of the zinc oxide-recognizing peptide of the present invention. Examples of such second protein include a gold-recognizing peptide having the sequence LKAHLPPSRLPS and gold-binding antibodies (JP Patent Publication No. 2005-312446 A and JP Patent Publication No. 2006-225294 A).

### Effect of the Invention

According to the present invention, an antibody required for detection of a target substance can be conveniently immobilized onto zinc oxide. This makes it possible to develop high-throughput sensing technology such as immunobiosensors.

### Brief Description of the Drawings

Fig. 1A schematically shows a VHH 1 or 2 or 3 expression vector, Fig. 1B schematically shows pTZ-VHH, and Fig. 1C schematically shows pRA 4F2-AuVHH1.
Fig. 2A shows a procedure for protein purification and Fig. 2B shows the results of confirming expression of VHH1, VHH2, and VHH3 in *Escherichia coli.*
Fig. 3 is a graph showing the results of gel filtration chromatography carried out for peptide-grafted antibodies (from the top in the graph, VHH2 (yellowish green), VHH3 (blue), WT VHH1 (black), and VHH1 (red)).
Fig. 4 is a graph showing the results of circular dichroism spectra measurement (from the top in the graph, WT VHH1 (black), VHH3 (blue), VHH1 (red), and VHH2 (yellowish green)).
Fig. 5 shows the process for evaluation of the binding of zinc oxide particles to peptide-grafted antibodies.
Fig. 6A shows the results of analyzing each supernatant, wash, and elution fraction shown in Fig. 5 by SDS-PAGE (1: Total, 2: Supernatant fraction, 3-5: Wash fraction, and 6: Elution fraction). Fig. 6B shows the results of tests for evaluation of material recognition by SDS-PAGE.
Fig. 7 shows the results of evaluating the binding activity of VHH3 to zinc oxide (in 1 mM phosphate buffer).
Fig. 8 shows an adsorption isotherm (A) and a Langmuir plot (B) for adsorption of VHH to zinc oxide.
Fig. 9 schematically shows protocols for constructing a peptide library wherein mutagenesis has been carried out for CDR H-3.
Fig. 10 shows the results of evaluating material recognition by SDS-PAGE.
Fig. 11 is a graph showing the results of gel filtration chromatography carried out for 4F2 (WT VHH1 (black), VHH1 (blue), and 4F2 (orange)).
Fig. 12 is a graph showing the circular dichroism spectra of 4F2 (WT VHH1 (black), VHH1 (blue), and 4F2 (orange)).
Fig. 13 shows photographs showing the results of evaluating the dispersibility of zinc oxide particles (A: antibody 3 µM and B: antibody 0.5 µM).
Fig. 14 shows the results of a dissociation test conducted for zinc oxide-binding antibodies immobilized on zinc oxide particles.
Fig. 15 schematically shows co-expression vectors for Fv(H-2), (H-3), (L-2), and (L-3).
Fig. 16 shows the results of CDR grafting of a zinc oxide-recognizing peptide to mouse-derived anti-hen egg white lysozyme antibody HyHEL-10 Fv. "A" shows the three-dimensional structure of HyHEL-10 Fv. "B" shows the gel filtration generation process of an antibody prepared by grafting of the peptide to the heavy chain CDR-2. "C" shows the results of circular dichroism spectra of VH alone (VH2sd) prepared by grafting the peptide to the heavy chain CDR-2.
Fig. 17A shows the results of evaluating the binding of mouse-derived anti-hen egg white lysozyme antibody Fv alone to zinc oxide (Fv (H-2): grafted into V-chain CDR-2, Fv (H-3): grafted into V-chain CDR-3, Fv (H-2): grafted into L-chain CDR-2, and Fv (L-3): grafted into L-chain CDR-3)). Fig. 17B also shows the results of evaluating the binding of purified peptide-grafted VH chain to zinc oxide.
Fig. 18 shows the results of evaluating the binding of zinc oxide-recognizing peptide fragments to zinc oxide.
Fig. 19 is a conceptual diagram of a biosensor using the zinc oxide-binding antibody of the present invention.
Fig. 20 shows the results of evaluating GFP immobilization using a flow system based on RlfS detection (concentration of protein flow: 10 mM, flow solvent:10 or 30 mM PO₄³⁻ (pH 7.5), 150 mM NaCl, flow rate: 10 ml/min, and saturated binding level: 89 nmol/m²).
Fig. 21 shows the results of evaluating immobilization of zinc oxide-binding camel antibody 4F2 on a flow system (saturated binding level: 130 nmol/m²).
Fig. 22 shows the results of evaluating immobilization of anti-GFP camel antibody 4F2 on a flow system (camel antibody 4F2 (lower): 134 nmol/m², anti-GFP camel antibody 4F2 (upper): 134 nmol/m², and amount of GFP immobilized: 110 nmol/m²).

This description includes part or all of the contents as disclosed in the description and/or drawings of Japanese Patent Application No. 2007-035073, from which the present application claims a priority.

### Best Mode for Carrying Out the Invention

### 1. Zinc oxide-recognizing peptide

Zinc oxide-recognizing peptides used in the present invention can specifically recognize zinc oxide and can bind to zinc oxide with high affinity. The present inventors have obtained 5 types of peptide capable of binding to zinc oxide: EAHVMHKVAPRP (ZnO1: SEQ ID NO: 1), QNTATAVSRLSP (ZnO2: SEQ ID NO: 2), ATHTNQTHALYR (ZnO3: SEQ ID NO: 3), VSNHKALDYPTR (ZnO4: SEQ ID NO: 4), and DSGRYSMTNHYS (ZnO5: SEQ ID NO: 5) by the screening using a phage display (see, JP Patent Publication No. 2006-225294 A). Among these peptides, the peptide of SEQ ID NO: 1 has binding activity to zinc oxide, as high as 10⁻⁷ M. The present inventors have further analyzed the sequence of the peptide, so as to specify that 7 amino acids consisting of EAHVMHK (SEQ ID NO: 6) is important for recognition of zinc oxide (see The 71^{st} Annual Meeting of the Society of Chemical Engineers, Japan, Lecture Summaries B206, Reference Example 1). In particular, histidine that is a basic amino acid has a significant effect on an association (binding) constant. Hence, the HVMH region between 2 histidine residues is thought to be highly likely important as a binding motif.

In the present invention, a peptide comprising the amino acid sequence having 7 amino acids of EAHVMHK can be used as a zinc oxide-recognizing peptide. Preferably, the zinc oxide-recognizing peptide may have a size of 7 to 20 amino acids and particularly has a size of approximately 7 to 12 amino acids, but examples of such size are not limited thereto.

In addition, each amino acid composing a peptide may be adequately modified or derivatized, as long as a desired binding activity to zinc oxide is maintained. Such a modified or derivatized peptide may also be included in the zinc oxide-recognizing peptide of the present invention.

### 2. Camel antibody

### 2.1 Characteristics of camel antibody

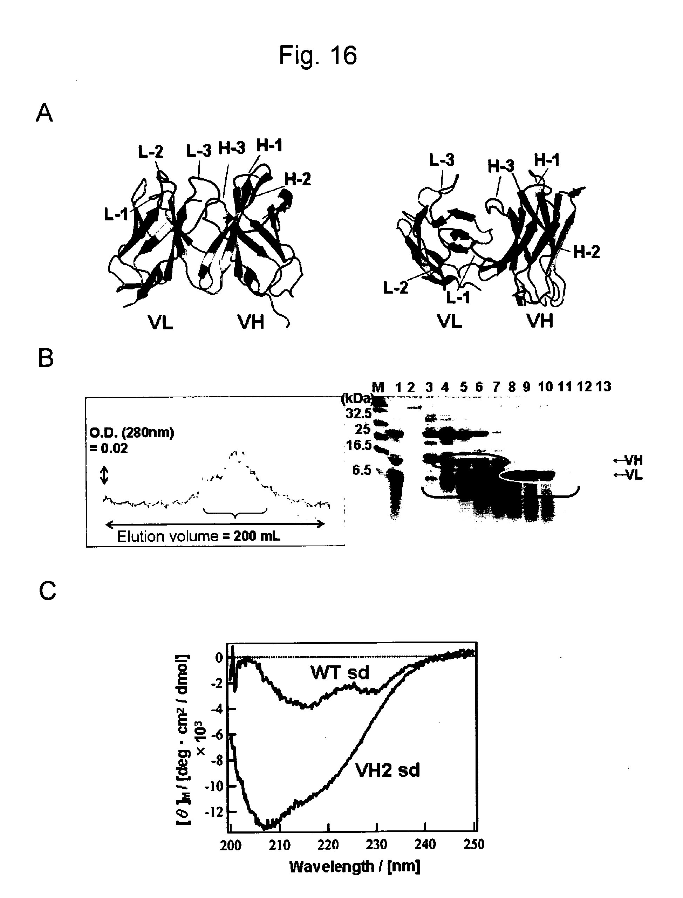

Antibodies used in the present invention are camel-derived antibody molecules (camel antibodies). In general, variable regions of an antibody form a heterodimeric structure composed of heavy chains and light chains. Fig. 16A shows the three-dimensional structure of mouse-derived anti-hen egg white lysozyme antibody HyHEL-10 Fv, which is a dimer composed of heavy chain (VH) and light chain (VL) subunits. However, a camel antibody has no light chain, but exerts antigen binding activity with its VH domain alone. Therefore, such a camel antibody has a molecular weight smaller than those of conventional antibodies and has a simple structure, so that it can be easily produced in large amounts using yeast or the like.

It has been thought that camel antibodies are not appropriate for peptide grafting into CDRs because they have no canonical structure and many of them have disulfide bonds between CDR H-1 and H-3 for stabilization of complementarity determining region (CDR) loops. However, in recent years, anti-β-lactamase camel antibody cAb BCII-10 has been isolated and designated "Universal VHH framework" (J. Mol. Biol. 352 (2005) 597), and may be suitable for CDR-grafting. The cAb BCII-10 has no disulfide bond between CDRs and the recombinant antibody can be expressed at high levels after CDR grafting. So, the cAb BCII-10 is extremely useful as a template antibody for peptide grafting. In the present invention, such camel antibody having no disulfide bond between CDRs is preferably used and the above cAb BCII-10 is a preferred example thereof.

The present inventors grafted a zinc oxide-recognizing peptide to the above-mentioned camel antibody CDR site and found that the antibody structure was not disrupted. When a conventionally-used mouse-derived anti-hen egg white lysozyme antibody having a heterodimeric structure was used, a fusion protein wherein a zinc oxide-recognizing peptide had been grafted into a CDR (CDR H-2) showed zinc oxide binding activity, but could not avoid any VH-VL dissociation phenomenon during its purification process (Fig. 16B). The dissociated peptide-grafted VH alone could not have the structure characteristic to antibodies (found to have a random structure: Fig. 16C) and lost antibody functions.

On the other hand, domains of a camel antibody are not associated, so that the domain dissociation (phenomenon) does not take place. Moreover, it was confirmed that a fusion protein wherein a zinc oxide-recognizing peptide had been grafted into its CDR exerted high binding activity to zinc oxide while stably maintaining the antibody structure.

Also, a camel antibody is a monomer having a small molecular weight. And, all molecules are linked via covalent bonds and dissociation (phenomenon) takes place. Accordingly, a camel antibody is advantageous in that a fusion protein can be easily prepared with other domains. With the use of this advantage, a different material-bound protein can also be prepared by fusing the zinc oxide-binding antibody of the present invention to another material-recognizing peptide or the like, as described below.

Stability of antibody structures is extremely important when the antibody is used for protein chips or biosensors described later. A camel antibody that is a monomer has extremely higher stability than that of conventional antibodies having dimeric structures, making it possible to provide a zinc oxide-binding antibody appropriate for practical uses.

### 2.2 Primary structure of camel antibody

The full-length amino acid sequence of a camel antibody is known. The sequence of cAb BCII-10 is described in Antimicrobial Agents and Chemotherapy, 2807-2812 (2001). The full-length amino acid sequence of the camel antibody cAb BCII-10 having no disulfide bond between the CDRs is shown in SEQ ID NO: 7 of the sequence listing. In this sequence, residues 26-38 correspond to the CDR H-1 region, residues 53-69 correspond to the CDR H-2 region, and residues 102-117 correspond to the CDR H-3 region.

### 3. Zinc oxide-binding antibody (camel antibody-zinc oxide-recognizing peptide fusion protein)

In the present invention, the above-mentioned zinc oxide-recognizing peptide is grafted into a CDR region (particularly, the CDR H-1 region) of a camel antibody to generate a zinc oxide-binding antibody (camel antibody-zinc oxide-recognizing peptide fusion protein).

### 3.1 CDR grafting

In an antibody molecule, an antigen-binding site is also referred to as a complementarity determining region (CDR) or a hypervariable region. For production of antibody drugs, CDR grafting by substituting only the CDR with a heterologous (mouse) region is used for preparation of a humanized antibody.

In the present invention, a zinc oxide-recognizing peptide is grafted into a camel antibody by CDR grafting. The present inventors determined three CDRs (H-1, H-2, and H-3) existing in a camel antibody by specifying the exposed regions on the CDR surface which is not directly linked to framework regions (residues 29-35, residues 53-69, and residues 102-117) based on the wild-type three-dimensional structure, and then grafted a zinc oxide-recognizing peptide into each of them.

### 3.2 Structural changes and zinc oxide binding activity after CDR grafting

The zinc oxide-binding antibody of the present invention must be able to maintain the original antibody structure even after CDR grafting and be capable of binding to zinc oxide to such a degree equivalent to or higher than that of the zinc oxide-recognizing peptide used for the grafting.

The three-dimensional structure of the above camel antibody-zinc oxide-recognizing peptide fusion protein can be examined by X-ray crystal structure analysis or measuring nuclear magnetic resonance spectra, fluorescence spectra, circular dichroism spectra, or the like. When the result for a wild-type camel antibody is completely different from that for a fusion protein, it is understood that grafting into the relevant site will alter the antibody structure and lower antibody activity.

The zinc oxide binding activity of a camel antibody-zinc oxide-recognizing peptide fusion protein can be evaluated by measuring association (binding) rate, dissociation rate, dissociation equilibrium constant, saturated binding level, and the like. For example, a dissociation equilibrium constant can be determined by adding zinc oxide particles to a buffer containing the fusion protein, collecting a supernatant, calculating the amount of zinc oxide bound to the fusion protein based on the amount of unadsorbed zinc oxide, and then carrying out Langmuir plotting. For convenient and detailed evaluation of binding properties, a method using surface plasmon resonance is preferred and details concerning the method are known in the art.

In the case of grafting into three CDRs (H-1, H-2, or H-3) carried out by the present inventors, it was confirmed that grafting into the H-2 region alters the structure characteristic to camel antibodies and causes the loss of the antibody activity. Also, the antibody that contains grafting into the H-1 region maintained binding activity (dissociation equilibrium constant (Kd) = 1.76 × 10⁻⁷ M) at a level equivalent to that of the zinc oxide-recognizing peptide before grafting. The antibody that contains grafting into the H-3 region exerted zinc oxide binding activity, but the binding activity was weaker than that of the antibody that contains grafting into the H-1 region.

The above results revealed that a zinc oxide-binding protein having high binding activity can be obtained by grafting a zinc oxide-recognizing peptide to the CDR H-1 region and particularly an exposed site on the antibody surface. Hereinafter, an antibody prepared by grafting a zinc oxide-recognizing peptide to the camel antibody CDR H-1 region is referred to as "VHH1."

### 4. Affinity maturation (high-affinity mutant) of fusion protein

The present inventors attempted to obtain a high-affinity mutant with enhanced binding activity using VHH1 prepared as in the previous section as a template. The H-2 region was determined to be necessary for stably maintaining the antibody structure based on the results of grafting into the three CDRs. Thus, the H-3 region was used as a target for mutant preparation.

### 4.1 In vitro affinity maturation

*In vitro* affinity maturation comprises two steps of: constructing a mutant library via mutagenesis; and screening the library for a high-affinity antibody. Mutagenesis can be carried out using a method known in the art, such as a chain shuffling, random mutagenesis, or CDR walking. Examples of a method for selection from the thus constructed mutant library include a method for selecting a high-affinity antibody using a low-concentration antigen, a method for collecting only an antibody strongly binding to an antigen under stringent washing conditions, and a method for selecting only a high-affinity antibody using an antagonistic reaction. The zinc oxide-binding antibody of the present invention is a low-molecular-weight single-stranded antibody, so that it can be easily produced in large amounts and the above steps of affinity maturation can also be easily carried out.

### 4.2 Preparation of high-affinity zinc oxide-binding antibody mutant

Based on previous examination, it was expected that an HXXH (H maybe R: SEQ ID NO: 8) motif flanked by basic amino acids is important for binding to zinc oxide. The 12 amino acids of HXXHXXHXXH (H may optionally be R, and X is G (glycine), L (leucine), R (arginine: SEQ ID NO: 9), or V (valine)) wherein HXXH was repeated 3 times was introduced into the above VHH1, and the binding activity to zinc oxide was evaluated. In addition, amino acid X within the motif was limited to a histidine, glycine, arginine, leucine, or valine residue in view of the fact that histidine, tryptophan, arginine, lysine, and glycine residues are important for binding to inorganic materials according to the sequence information reported so far of inorganic material-binding peptides and in view of a library scale theoretically calculated from codons encoding amino acids and a library scale that can be constructed.

As a result, 4 clones: HLGHGGHRLH (SEQ ID NO: 10), HLGHGGHGLH (SEQ ID NO: 11), HVGHGLHGVR (SEQ ID NO: 12), and HLGHGLHRVH (SEQ ID NO: 13) having an affinity for zinc oxide superior to that of VHH1 were selected. The dissociation equilibrium constants (Kd) of all of these clones were far lower than that (1.76 × 10⁻⁷M) of VHH1 when each saturated binding level was equivalent to that of VHH1. Particularly, 4F2 was found to have excellent selectivity such that it had extremely high affinity (dissociation equilibrium constant (Kd) = 9.39 × 10⁻⁹ M) for zinc oxide, but showed almost no affinity for other materials. It was thus confirmed that the three-dimensional structure of the 4F2 antibody was appropriately maintained. The full-length amino acid sequence of 4F2 is shown in SEQ ID NO: 14 of the Sequence Listing.

### 5. Expression vector for zinc oxide-binding antibody

The present invention also provides a vector that contains a gene encoding the above zinc oxide-binding antibody and can express the zinc oxide-binding antibody.

The expression vector of the present invention can be constructed by ligating (inserting) a gene encoding the zinc oxide-binding antibody of the present invention to a known vector such as a plasmid. A nucleotide sequence may be appropriately optimized depending on host cells for introduction. Vectors are not particularly limited, as long as they can be replicated within hosts. For example, plasmid DNA, phage DNA, and the like can be used.

Examples of the above plasmid DNA include plasmids from *Escherichia coli* (e.g., pBR322, pBR325, pUC18, pUC119, pTrcHis, and pBlueBacHis), plasmids from *Bacillus subtilis* (e.g., pUB110 and pTP5), and plasmids from yeast (e.g., YEp13, YEp24, YCp50, and pYE52). An example of phage DNA is λphage.

Insertion of a gene into the above vector can be carried out by cleaving purified DNA with appropriate restriction enzymes and then inserting the cleaved DNA into an appropriate restriction site or a multicloning site of vector DNA in order to ligate it to the vector.

An appropriate promoter should be placed upstream of the structural gene to express a foreign gene within a host. Any promoter known to function within hosts can be used as the promoter and not particularly limited. Regarding promoters, promoters for each host will be described in detail in the next section. Also, if necessary, a *cis* element such as an enhancer, a splicing signal, a polyA addition signal, a ribosome binding sequence (SD sequence), a terminator sequence, and the like may also be placed.

The vector may also contain a gene encoding other enzymes or proteins that are active in host cells. As described later, the zinc oxide-binding antibody of the present invention can also be expressed as a fusion protein with another material-recognizing peptide. In this case, a gene encoding a fusion protein may be introduced into the vector.

### 6. Recombinant production of zinc oxide-binding antibody

The zinc oxide-binding antibody of the present invention can be recombinantly produced by introducing the above-described expression vector into appropriate hosts in a condition that the gene can be expressed and then culturing the hosts. In particular, the zinc oxide-binding antibody of the present invention is generated based on a camel antibody, and is a low-molecular-weight monomeric antibody having no disulfide bond, so that it can be efficiently produced by recombination. High production of cAb BCII-10 using yeast has been confirmed (*supra*).

Examples of host cells include any cells, as long as they are prokaryotic cells, such as *Escherichia coli* and *Bacillus subtilis*. For transformation of these host cells with a gene of interest, a host cell can be transformed with a plasmid vector containing a replicon that is derived from species compatible with the host; that is, a replication origin and a regulatory sequence. As the vector, a vector having a sequence capable of conferring phenotype selectivity to transformed cells is preferred.

For example, in the case of *Escherichia coli*, strains including the K12 strain are often used. As a vector, generally a pBR322 or pUC-based plasmid is used. However, strains and vectors are not limited thereto and various known strains or vectors can be used. Also, examples of promoters used for *Escherichia coli* include a tryptophan (trp) promoter, a lactose (lac) promoter, a tryptophan-lactose (tac) promoter, a lipoprotein (lpp) promoter, and a polypeptide chain elongation factor Tu (tufB) promoter. All of these promoters can be appropriately used.

Also, in the case of *Bacillus subtilis*, the 207-25 strain is preferred. As a vector, pTUB228 (Ohmura, K. et al. (1984) J. Biochem. 95, 87-93) can be used, but vectors are not limited thereto. In addition, a DNA sequence encoding a signal peptide sequence of α-amylase from *Bacillus subtilis* can be linked to a vector so that a protein can be expressed and secreted from microorganisms (cells).

Examples of eukaryotic cells to be used as host cells include vertebrate cells, insect cells, and yeast cells. As vertebrate cells, for example, dihydrofolate reductase-deficient lines derived from monkey COS cells (Gluzman, Y. (1981) Cell 23, 175-182, ATCC CRL-1650) and Chinese hamster ovary cells (CHO cells, ATCC CCL-61) (Urlaub, G. and Chasin, L. A. (1980) Proc. Natl. Acad. Sci. U.S.A. 77, 4126-4220) are often used, but not limited thereto.

As an expression vector for vertebrate cells, generally a vector comprising a promoter located upstream of a gene to be expressed, a splice site of RNA, a polyadenylation site, a transcription termination sequence, and the like can be used. If necessary, such an expression vector has a replication origin. Examples of the expression vector include, but are not limited to, pCR3.1 (Invitrogen) having a cytomegalovirus early promoter and pSV2dhfr having an SV40 early promoter (Subramani, S. et al. (1981) Mol. Cell. Biol. 1, 854-864).

When COS cells are used as host cells, for example, an expression vector that can be preferably used has an SV40 replication origin, can undergo autonomous replication in COS cells, and comprises a transcription promoter, a transcription termination signal, and an RNA splice site. Such expression vector can be introduced into COS cells by a diethylaminoethyl (DEAE)-dextran method (Luthman, H. and Magnusson, G. (1983) Nucleic Acids Res, 11, 1295-1308), a calcium phosphate-DNA co-precipitation method (Graham, F. L. and van der Eb, A. J. (1973) Virology 52, 456-457), an electroporation (Neumann, E. et al. (1982) EMBO J. 1, 841-845), or the like. Thus, desired transformed cells can be obtained.

Also, when CHO cells are used as host cells, transformed cells stably producing a polypeptide of interest can be obtained by co-transfecting the expression vector and a vector capable of expressing a neo gene that functions as an antibiotic G418-resistant marker, such as pRSVneo (Sambrook, J. et al. (1989): "Molecular Cloning A Laboratory Manual" Cold Spring Harbor Laboratory, NY) or pSV2neo (Southern, P. J. and Berg, P. (1982) J. Mol. Appl. Genet. 1, 327-341) into CHO cells, and then selecting G418-resistant colonies.

When insect cells are used as host cells, an ovary cell-derived established cell line (Sf-9 or Sf-21) of *Spodoptera frugiperda* of the family Noctuidae of the class Lepidoptera, egg cell-derived High Five cells of *Trichoplusia ni* (Wickham, T. J. et al, (1992) Biotechnol. Prog.i: 391-396), and the like are often used as host cells. As a baculovirus transfer vector, pVL1392/1393 using the polyhedrin protein promoter of *Autographa californica* nuclear polyhedrosis virus (AcNPV) is often used (Kidd, i. M. and V.C. Emery (1993) The use of baculoviruses as expression vectors. Applied Biochemistry and Biotechnology 420, 137-159). In addition to these examples, a vector using baculovirus P10 or a promoter of a similar basic protein can also be used. Furthermore, a recombinant protein can be expressed as a secretory protein by ligating a secretory signal sequence of AcNPV envelope surface protein GP67 to the N-terminus of a protein of interest (Zhe-mei Wang, et al. (1998) Biol. Chem., 379, 167-174).

As an expression system using eukaryotic microorganisms as host cells, generally yeast is known well. Particularly, yeast of the genus *Saccharomyces*, such as bread yeast *Saccharomyces cerevisiae* or petroleum yeast *Pichia pastoris* may be preferred. For expression vectors for eukaryotic microorganisms such as yeast, for example, an alcohol dehydrogenase gene promoter (Bennetzen, J. L. and Hall, B. D. (1982) J. Biol. Chem. 257, 3018-3025), an acid phosphatase gene promoter (Miyanohara, A. et al. (1983) Proc. Natl. Acad. Sci. U.S.A. 80, 1-5), and the like can be preferably used. Also, when expressed as a secretory protein, it can be expressed in the form of a recombinant protein having a secretory signal sequence and endogenous protease of host cells or a cleavage site of known protease on the N-terminus. For example, it is known that, in a system wherein human mast cell tryptase (trypsin-type serine protease) is expressed in petroleum yeast, a secretory signal sequence of yeast α factor and a cleavage site of petroleum yeast KEX2 protease are fused to the N-terminus and then expressed, so that activated tryptase is secreted in culture medium (Andrew, L. Niles, et al. (1998) Biotechnol. Appl. Biochem. 28, 125-131).

Transformants obtained as described above can be cultured according to a conventional method, so that the zinc oxide-binding antibody of interest can be produced within or outside the cultured cells. As culture media used for such culturing, various culture media can be appropriately selected from conventional culture media depending on host cells employed. For example, when the above COS cells are host cells, culture media such as an RPMI1640 medium, Dulbecco's modified eagle medium (hereinafter, referred to as "DMEM"), or these media optionally supplemented with a serum component such as fetal calf serum can be used.

A recombinant protein that is produced within or outside transformed host cells as a result of culturing can be separated and purified by a known separation technique based on the physical properties, chemical properties, or the like of the protein. Examples of such method include treatment with the use of a protein precipitation agent, ultrafiltration, molecular sieve chromatography (gel filtration), adsorption chromatography, ion exchange chromatography, affinity chromatography, various types of liquid chromatography such as high performance liquid chromatography (HPLC), and dialysis. These methods can be used independently or in combination. Also, recombinant protein ligated to 6 histidine residues can be expressed and efficiently purified on a nickel affinity column. A zinc oxide-binding antibody protein of interest can be easily produced with a high yield and high purity through the use of appropriate combination of the above-described methods.

### 7. Fusion of zinc oxide-binding antibody and second protein

As described above, a camel antibody is a monomeric antibody having a small molecular weight, wherein all molecules are each bound via covalent bonds, so that no dissociation phenomenon takes place. Therefore, a recombinant protein can be easily prepared by fusing the zinc oxide-binding antibody of the present invention to another protein. The present invention also provides such a fusion protein.

Such a fusion protein can be prepared with reference to previous reports (Nature Biotech. 23, 1126-1136./Cancer Immunol. Immunother., 53, 497-509 (2004), Biochem. Biophys. Res. Commun., 328, 98-105 (2005)).

If a protein containing another material-recognizing peptide (second protein having a material-recognizing sequence) is used as a protein to be fused, the thus obtained fusion protein plays a role in linking two different materials. In addition, the term "second protein having a material-recognizing sequence" refers to a protein that contains a sequence specifically recognizing and binding to a material (e.g., metal or metal oxide), as in the case of the zinc oxide-recognizing peptide of the present invention. Many such proteins are known in the art, such as those described in WO2004/031381, WO2005/010031, WO2006/064639, WO2006/068250, WO2006/126595, Brown et al., J. Mol. Biol. (2000), 299, 725-735, Naik et al., Nature materials, (2002), Vol.1, November, 169-172, Naik et al., J. Nanosci. Nanotech., (2002), Vol.2, No.1, 95-100, Mao et al., PNAS, (2003), Vol.100, No. 12. 6946-6951, and Mao et al., Science, (2004), Vol.303, 213-217. These proteins can be appropriately used. Such fusion proteins can be used for array technology for nanoparticles, technology for binding nanoparticles, and the like.

For example, a fusion protein prepared by fusing a gold material-recognizing peptide (LKAHLPPSRLPS: SEQ ID NO: 20) or a gold-binding antibody (JP Patent Publication No. 2005-312446 A and JP Patent Publication No. 2006-225294 A) to the zinc oxide-binding antibody of the present invention makes it possible to immobilize zinc oxide particles on the surfaces of gold materials and to bind zinc oxide particles to gold nanoparticles.

In addition to the above examples, the zinc oxide-binding antibody of the present invention can be applied for protein purification by fusing a His tag peptide, a GST protein, or various binding proteins thereto.

### 8. Zinc oxide-binding antibody-immobilized support (protein chip and nanoparticle)

The present invention provides a solid support containing a zinc oxide layer on which the zinc oxide-binding antibody has been immobilized. The shape of such support is not particularly limited, as long as it contains a zinc oxide layer. The support may be in the form of a substrate (e.g., a protein chip) or a particle (e.g., a nanoparticle).

### 8.1 Protein chip

It is desired to achieve the following conditions for preparation of a protein chip that enables "stable," "convenient," and "rapid" measurement: (1) proteins are difficult to be adsorbed; (2) proteins can be selectively immobilized; and (3) patterning is easily carried out on a substrate.

Examples of general methods for immobilizing proteins on substrates include: (a) an immobilization method using gold-sulfur bonding onto a gold substrate; (b) an immobilization method using bonding to a silica film with the use of a silane coupling agent; (c) an immobilization method using chemical bonding that uses a functional group displayed on an organic film bound via the method of (a) or (b) above. However, these techniques are problematic in that protein immobilization results in lowered protein activity, processing of a substrate is complicated; and a protein immobilization method comprises multiple steps, for example.

Zinc oxide is ceramic, so that proteins are difficult to be adsorbed onto zinc oxide. By the use of the zinc oxide-binding antibody (camel antibody) according to the present invention, proteins can be conveniently and rapidly immobilized on a zinc oxide surface. Also, in the case of zinc oxide, substrates with various structures can be produced through the use of semiconductor processing technology (microprocessing technology). Furthermore, as in previous reports, patterning technology therefor has been established (JP Patent Publication No. 2006-225294 A). The zinc oxide-binding antibody according to the present invention may be useful for producing solid substrates such as protein chips.

In addition, the material of such solid substrate is not particularly limited, as long as a zinc oxide layer can be formed. Substrates that are generally used in the art, such as metal, glass, and silicon substrates, can be appropriately used.

### 8.2 Nanoparticle/Microparticle

Particles (nano size or micro size) having zinc oxide layers on which the zinc oxide-binding antibody of the present invention has been immobilized can be applied for various analyses such as protein purification. Particularly nanoparticles are useful with their surface areas for high-sensitive analyses within microchannels.

Also, zinc oxide-binding antibody-immobilized zinc oxide particles exert good dispersibility in an aqueous solution because the particles are modified with the protein and therefore, they efficiently react with an active substance. When complete dispersion is achieved, particles are aggregated upon protein-to-protein interactions. Thus, binding between proteins can be conveniently analyzed. In addition, such good dispersibility is also important for patterning of nanoparticles by an ink-jet technique or a spray technique.

### 9. Biosensor

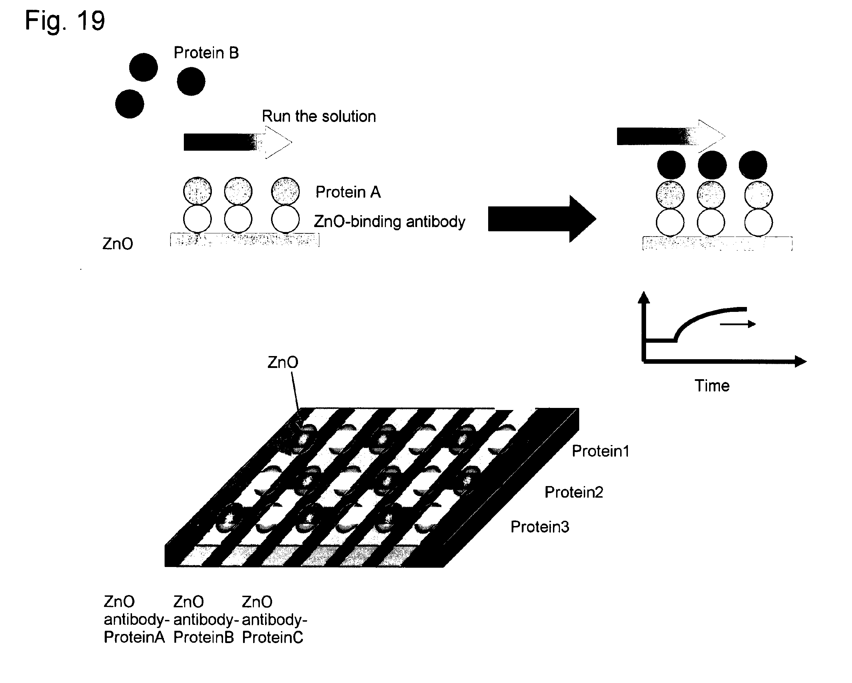

The present invention also provides a biosensor (immunobiosensor) having a solid support that contains a zinc oxide layer on which the zinc oxide-binding antibody has been immobilized and a means for detecting intermolecular interactions via the above antibody. The term "intermolecular interactions via (the above) antibody" refers not only to antibody-antigen interactions, but also to all antibody-mediated associations (binding) and dissociations with analytes, such as an interaction between an antibody and an antigen via a second antibody. The means fro detection may be any known detection means, such as an electric detection means (e.g., FET) and optical detection means such as surface plasmon resonance. Representative embodiments will be as described below. Fig. 19 is a conceptual diagram of a biosensor produced using the zinc oxide-binding antibody of the present invention.

### 9.1 Biosensor using FET technology

Field-Effect Transistor (FET) technology is generally applied for biosensors using electric detection. The principle thereof is as follows: a current flowing from a source electrode to a drain electrode is controlled by a gate electrode (third electrode). A protein-to-protein interaction caused to take place on the surface of the gate electrode results in a change in the charge of the gate electrode; that is, a change in electric response (Anal. Chim. Acta 136, 93 (1982)). With the use of zinc oxide for such a gate electrode, a protein-to-protein interaction can be measured using FET technology. Hence, this can be used as a biosensor.

For detection of a protein-to-protein interaction via FET, it is necessary for the reaction to take place at a location as close as possible to the surface of a gate electrode. In the case of protein immobilization using the camel antibody 4F2 as a binding unit, a protein of interest can be immobilized at a position extremely close to a gate electrode. Thus, the camel antibody 4F2 is useful for FET with higher sensitivity than that of conventional technology and a biosensor using FET.

### 9.2 Biosensor using the surface plasmon resonance

A surface plasmon resonance sensor is a biosensor capable of analyzing in real time intermolecular interactions on a sensor chip with the use of surface plasmon resonance. A commercially available surface plasmon resonance sensor (e.g., a gold film sensor chip for SPR measurement, Sensor Chip Au (BIAcore)) is already easily available. A zinc oxide layer is formed on a sensor chip, so that the zinc oxide-binding antibody of the present invention can be adsorbed stably with its high binding capability. A microchannel is provided on the thus prepared sensor chip onto which the zinc oxide-binding antibody has been bound, and then a sample containing an analyte (antigen) is loaded at a given flow rate through the microchannel. If the antibody interacts with the antigen in the sample, association (binding) and dissociation can be optically detected based on surface plasmon phenomena and detected sensorgram data can be monitored in real time. Such a surface plasmon resonance sensor is advantageous in that a trace amount of a sample can be detected with high sensitivity and the kinetics of interactions can be analyzed in real time. Therefore, a surface plasmon resonance sensor to which the zinc oxide-binding antibody of the present invention has been bound is useful as a biosensor for analysis of antigen-antibody interactions.

Furthermore, the zinc oxide-binding antibody of the present invention can also be applied to microprocessing of zinc oxide or two-dimensional surface plasmon resonance (SPR) using zinc oxide dots (each approximately several hundred µm). A zinc oxide film can be easily formed on an inorganic material (semiconductor material) such as micro-processed zinc with the use of semiconductor production technology. For example, this can be achieved by any technique such as a lift-off method, a sputtering technique using masking, or the like, as long as patterning can be carried out at the µm level. Furthermore, patterning using a Pd catalyst at room temperature (Adv. Mater. 14, 418-421 (2002)) is also possible.

### 9.3 Biosensor using reflectometry

Reflectometry is a technique for measuring the amount of a protein adsorbed onto the surface of a sample plate. It involves reflecting a laser off of the sample plate and then measuring reflected light. A reflectometry biosensor produced with a combination of the principle of such reflectometry and a microfluidic chip is easily available (e.g., a reflectometry biosensor array system Fluid-RIfS (Fluidware Technologies Inc)). A zinc oxide layer is formed on a sensor chip, so that the zinc oxide-binding antibody of the present invention is stably adsorbed with its high binding capability. A microchannel is provided on the thus prepared sensor chip onto which the zinc oxide-binding antibody has been bound, and then a sample containing an analyte (antigen) is loaded at a given flow rate through the microchannel. If the antibody interacts with the antigen in the sample, the amounts of the adsorbed protein can be monitored in real time in the form of sensorgram data. Such a reflectometry biosensor is advantageous in that a trace amount of a sample can be detected with high sensitivity and the kinetics of such interactions can be analyzed in real time. Therefore, a reflectometry biosensor to which the zinc oxide-binding antibody of the present invention has been bound is useful as a biosensor for analysis of antigen-antibody interactions.

Applications of the zinc oxide-binding antibody of the present invention are not limited to the above examples. The zinc oxide-binding antibody can also be applied to biosensors using transmitted beam, reflected light, near-field light, crystal oscillator microbalance of electromagnetic waves (e.g., infrared ray). Also, biosensors can also be developed, to which other examples of known technology employed for immunodetection (e.g., solid-phase immunoassay such as immunoprecipitation, a Western blot method, a dot blot method, a slot blot method, an ELISA method, and an RIA method) are appropriately applied.

### 10. Others

The zinc oxide-binding antibody of the present invention immobilized on a zinc oxide substrate can be easily dissociated by setting appropriate conditions. Specifically, such immobilized zinc oxide-binding antibody can be collected and analyzed by varying the conditions. Upon that, it should be noted that a solution to be used for dissociation may be a conventional aqueous solution for protein preparation (e.g., phosphate buffer) and a protein can be dissociated in a nondenatured form such that it keeps its active structure nondenatured.

### Examples

### Example 1: Preparation of zinc oxide-binding camel antibody

### 1. Grafting of ZnO-recognizing peptide to camel antibody

### (1) Camel antibody cAb BCII-10

A camel antibody originally has a disulfide bond between CDR H-1 and CDR H-3 for stabilization of complementarity determining region (CDR) loops. In this Example, an anti-β-lactamase camel antibody cAb BCII-10 (J. Mol. Biol. 352 (2005) 597) designated "Universal VHH framework" having no disulfide bond between CDRs was used as a template antibody for peptide grafting. cAb BCII-10 was prepared based on a sequence given in the previous report (Antimicrobial Agents and Chemotheraphy, 2807-2812 (2001)).

### (2) Construction of expression vector for cAb BCII-10 and peptide-grafted camel antibody

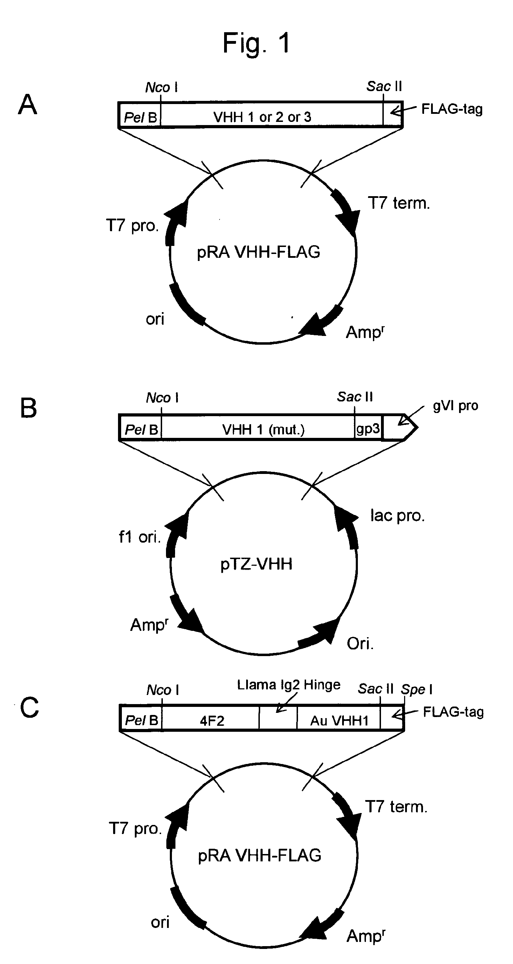

A gene encoding the antibody VHH3 in which a zinc oxide-recognizing peptide (SEQ ID NO: 1) comprising EAHVMHKVAPRP had been grafted into the 103-114 site of the CDR H-3 region of the anti-β-lactamase antibody cAbBCII-10 was fully synthesized using an overlap extension PCR method. Subsequently, wild-type cAb BCII-10 (WT VHH) was prepared using primers for substitution of CDR H-3 composed of the ZnO-recognizing peptide sequence with wild-type CDR. Similarly, zinc oxide-recognizing peptide was grafted into CDR H-1 and H-2, so that peptide-grafted camel antibodies VHH1 and VHH2 were prepared. Regarding positions for grafting into CDRs, 12 exposed amino acid residues (29-35 residues, 53-69 residues, and 102-117 residues) on the surfaces of CDRs were selected based on the three-dimensional structure of the wild-type camel antibody. Fig. 1A schematically shows the thus constructed expression vector. Also, the composition of a PCR reaction solution and reaction conditions (Table 1), PCR primers used and the amino acid sequences of the peptide-grafted camel antibodies (Table 2) are as shown below.

**[Table 1]**

| **« Composition of PCR reaction solution »** | | **« PCR reaction conditions »** | | | |
|---|---|---|---|---|---|
| Template | 1ml | denaturation | 94 °C | 30 sec | |
| primer (10pmol/ml) | each 5ml | annealing | 52 °C | 1 min | 30 cycle |
| 10×PCR buffer for LA Taq | 5ml | extension | 72 °C | 2 min | |
| dNTP 2mM each | 8ml | | | | |
| 25mM MgCl₂ | 5ml | | | | |
| LA Taq polymerase | 0.5ml | | | | |
| ddH₂O | 20.5ml | | | | |
| Total | 50ml | | | | |

### Primers for full synthesis of VHH3

VHH3-NcoI (Reverse) 5'-NNNCCATGGCCCAGGTTCAGCTGGTTGAAAG-3' (SEQ ID NO: 21) 
VHH3-1 (Reverse) 5'-CAGGTTCAGCTGGTTGAAAGCGGCGGCAGCGTGCAGG
CGGGCGGCAGCCTGCGCCTGAGCTGCACCGCGAGCGGCGGCAGCGAATATAGCTA
T-3'(SEQ ID NO: 22)
 
VHH3-2 (Forward) 5'-AATTGCTGCCACTGCTTCGCGTTCCTGGCCCGGCGCCTGGC
GAAACCAGCCCAGGCTAAAGGTGCTATAGCTATATTCGCTGCCGCC-3' (SEQ ID N
O: 23)
 
VHH3-3 (Reverse) 5'-GCGAAGCAGTGGCAGCAATTGCGAGCATGGGCGGCCTGACC
TATTATGCGGATAGCGTGAAAGGCCGCTTTACCATTAGCCGCGATAACGCGAAAAA
C-3' (SEQ ID NO: 24)
 
VHH3-4 (Forward) 5'-GCTTCCACTGCTGCGCAATAATAAATCGCGGTATCTTCCGGT
TTCAGGTTGTTCATCTGCAGGGTCACGGTGTTTTCGCGTTATCGCGGC-3' (SEQ ID
NO: 25)
 
VHH3-5 (Reverse) 5'-TATTGCGCAGCAGTGGAAGCGCATGTGATGCATAAAGTGGCG
CCGCGCCCGTTTCGCTATTGGGGCCAGGGCACCCAGGTGACCGTGAGCAGC-3' (SE
Q ID NO: 26)
 VHH3-sacII (Forward) 5'-NNNCCGCGGATGAAACGGTAACCTGGG-3' (SEQ ID NO: 27)

### Primers for synthesis of WTVHH

WTVHH (Forward) 5'-GCTCGGCAGGCGCATAAAATAGCCGCGCACTGCTGCGCAAT
AATAAATCG-3' (SEQ ID NO: 28)

WTVHH (Reverse) 5'-TATTTATGCGCCTGCCGAGCAGCCATAACTTTCGCTATTGG
GGCCAGGG-3' (SEQ ID NO: 29)

### Primers for preparation of VHH1, VHH2 and ZnOtag-WTVHH

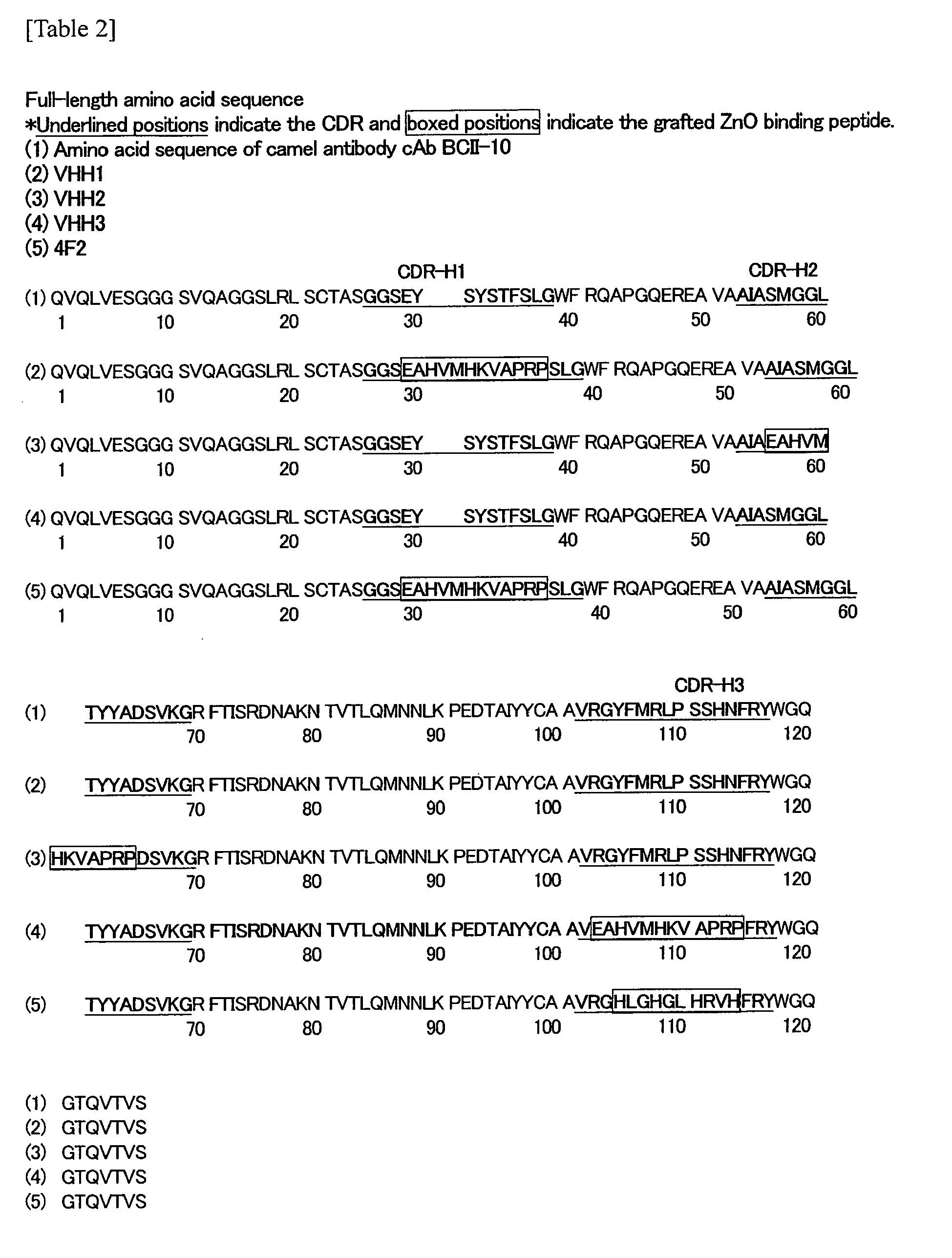

VHH-1 (Forward) 5'-GCGCCACTTTATGCATCACATGCGCTTCGCTACCACCGCTCGC
GGTGCAGCTCAG-3' (SEQ ID NO: 30)

VHH-1 (Reverse) 5'-TGTGATGCATAAAGTGGCGCCGCGTCCGAGCCTGGGCTGGTT
TCGCCAG-3' (SEQ ID NO: 31)

VHH-2 (Forward) 5'-GCGCCACTTTATGCATCACATGCGCTTCCGCAATTGCTGCCAC
TGCTTC-3' (SEQ ID NO: 32)

VHH-2 (Reverse) 5'-TGTGATGCATAAAGTGGCGCCGCGTCCGGATAGCGTGAAAGG
CCGCTTT-3' (SEQ ID NO: 33)

ZnOtag-VHH (B) 5'-NNNCCATGGCCGAAGCGCATGTGATGCATAAAGTGGCGCCGC
GTCCGCAGGTTCAGCTGGTTGAAAG-3' (SEQ ID NO: 34)

### 2. Expression of zinc oxide-recognizing peptide-grafted camel antibodies VHH1, VHH2, and VHH3 in Escherichia coli and evaluation of the structures and functions thereof

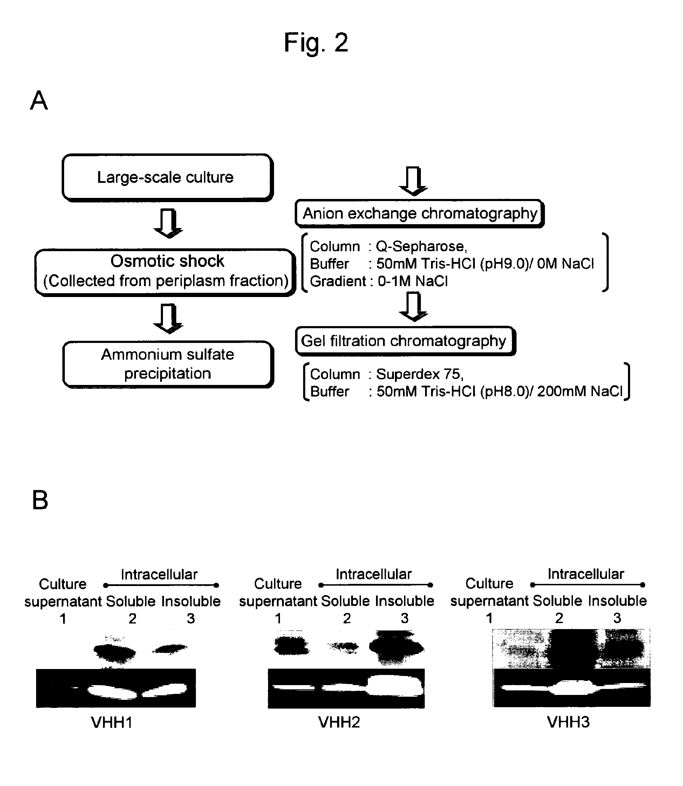

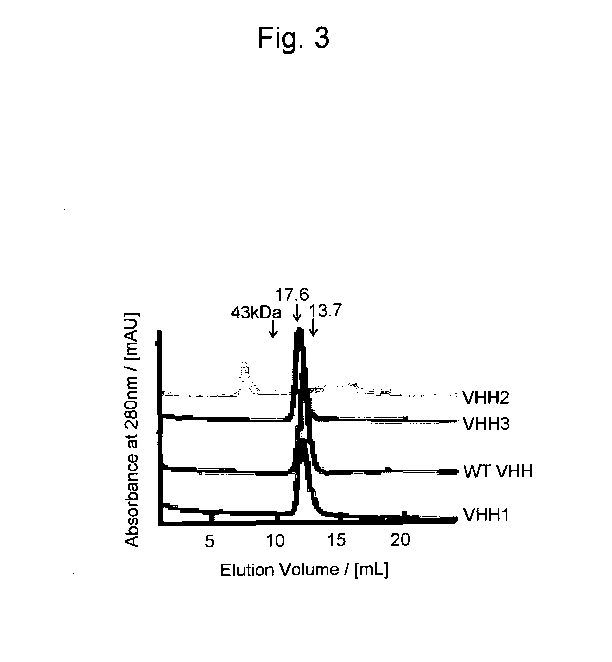

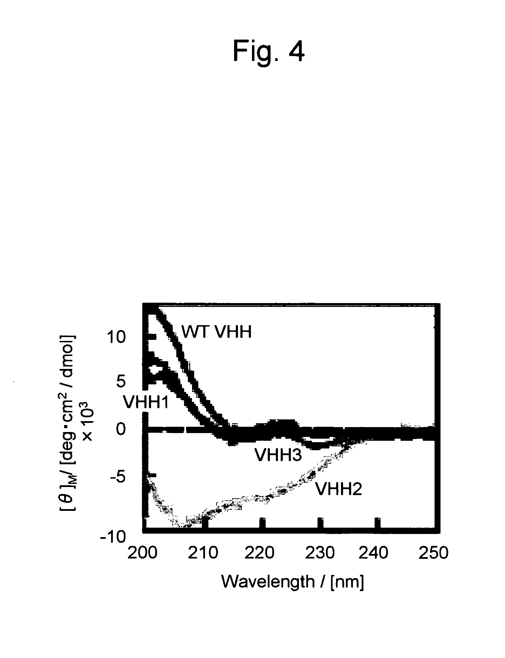

*Escherichia coli* BL21 (DE3) was transformed with the above-prepared VHH1, VHH2, and VHH3 expression vectors, and then the proteins were expressed. Fig. 2A shows a protein purification method. VHH1, VHH2, and VHH3 could all be prepared as soluble fractions (Fig. 2B). The thus prepared proteins were evaluated by gel filtration chromatography (Superdex75; 50 mM Tris-HCl (pH 8.0)/200 mM NaCl) and circular dichroisms (AVIV circular dichroism spectrometer (Proterion Co., New Jersey, U.S.A.); path length, 1.0 mm; resolution, 0.2 nm; average time, 4 s). Whereas VHH1 and VHH3 were each found to be monomeric single components (red and blue lines in Fig. 3) exerting circular dichroisms characteristic to camel antibodies (red line and blue line in Fig. 4), VHH2 showed no uniform gel filtration chromatogram and no such circular dichroisms characteristic to camel antibodies (yellowish green lines in Figs. 3 and 4).

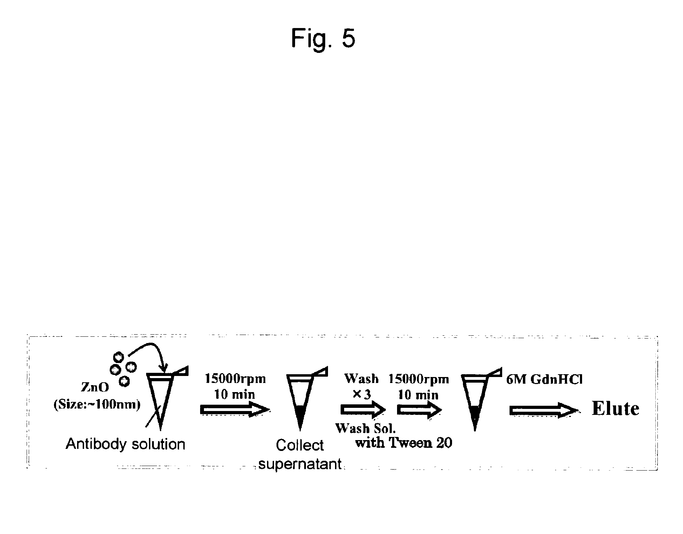

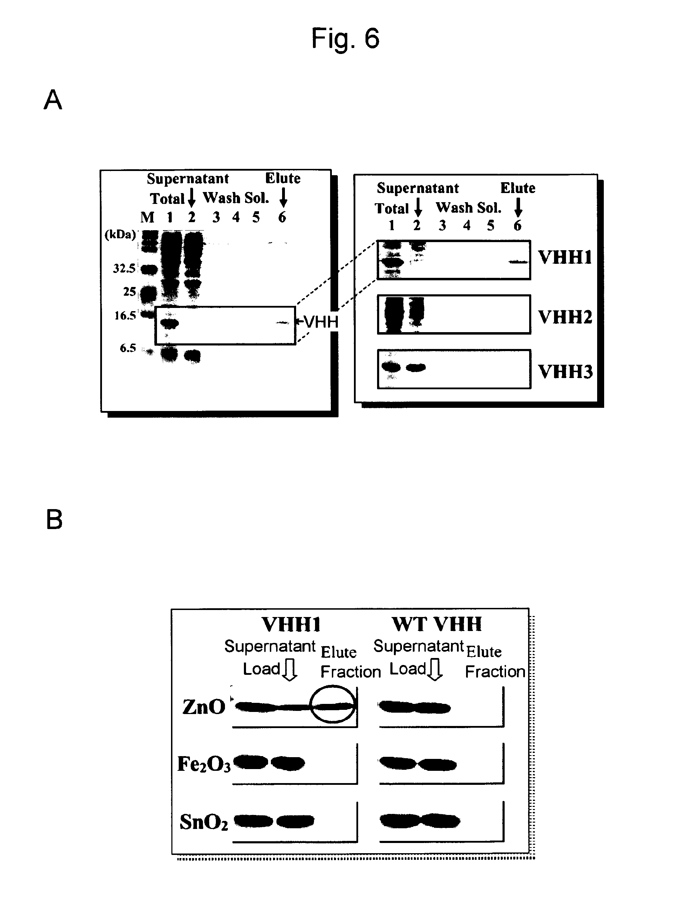

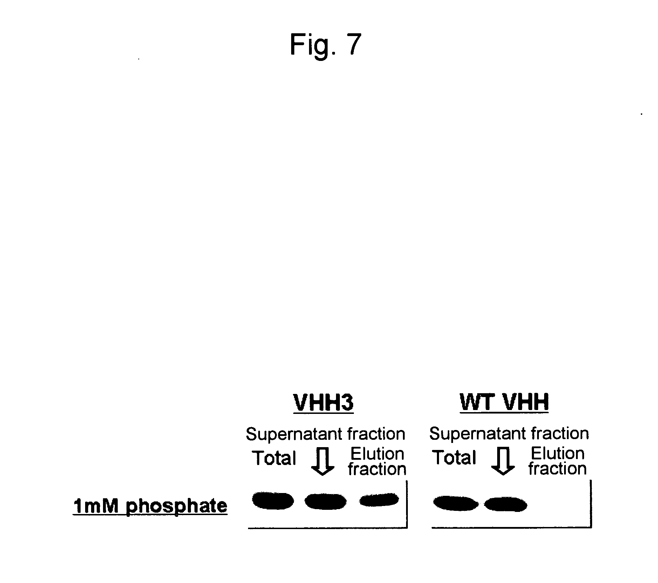

Next, activity of the prepared VHH1, VHH2, and VHH3 with respect to zinc oxide particles was evaluated. Specifically, these proteins and zinc oxide with a particle size of 100 nm were mixed in a 10 mM phosphate buffer (containing a surfactant) and then centrifuged, so as to separate supernatants from precipitates (zinc oxide) (Fig. 5). Precipitates were washed several times with the above phosphate buffer. Finally, adsorbed proteins were solubilized using an aqueous 6M guanidinium hydrochloride (GdnHCl) solution and then evaluated by SDS-PAGE (Fig. 6). The results of SDS-PAGE revealed that VHH1 bound to zinc oxide (Fig. 6A) while maintaining material specificity (Fig. 6A). Regarding VHH3, no binding properties were observed upon the use of a 10 mM phosphate buffer, but binding to ZnO upon the use of a 1 mM phosphate buffer (Fig. 7) was observed. Accordingly, VHH3 also had the function of binding to ZnO, but the binding activity was weaker than that of VHH1.

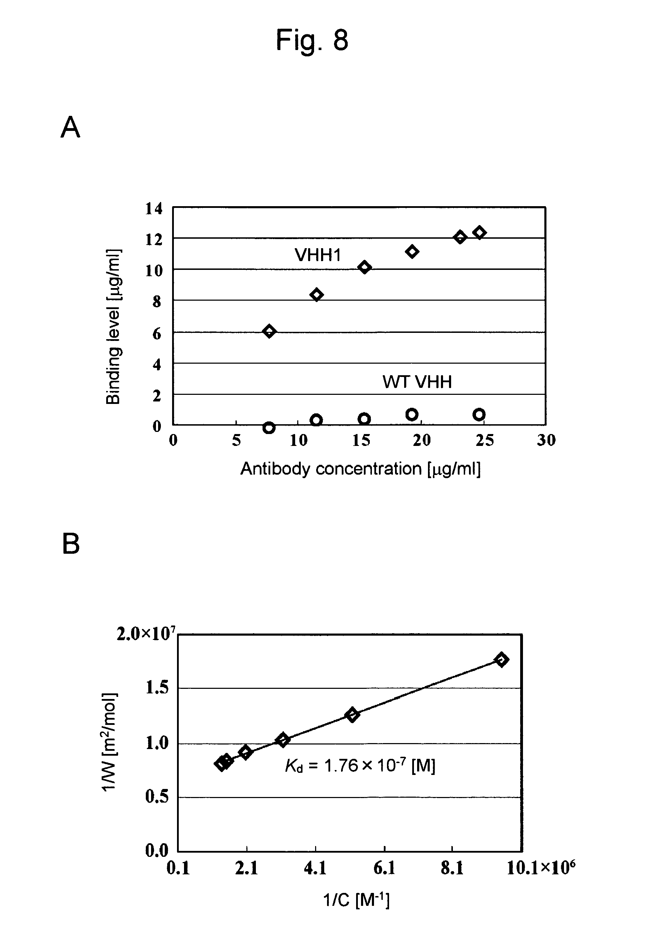

Moreover, the binding of VHH1 was evaluated using a Langmuir plot (Fig. 8). In this case, VHH1 had a dissociation equilibrium constant of 176 nM for zinc oxide. Hence, the activity of VHH1 was evaluated to be the same degree as that of a construct prepared by fusing a zinc oxide-recognizing peptide to the N-terminus of WT VHH (3.30 × 10⁻⁷ M). It was thus confirmed that a camel antibody's own three dimensional structure was maintained and the peptide activity was not lowered even after peptide grafting into the CDR H-1 region.

### Example 2: Preparation of high affinity camel antibody by in vitro affinity maturation

Further improvement of activity was attempted using VHH1 prepared in Example 1 as a template. Specifically, the CDR H-3 region to which no zinc oxide-recognizing peptide had been grafted was selected and then the amino acid sequence thereof was randomized to construct a library. The CDR H-2 region was not used for alteration, since it was found based on the results of Example 1 that when the sequence of this portion is altered, the camel antibody is structurally altered to lose its activity.

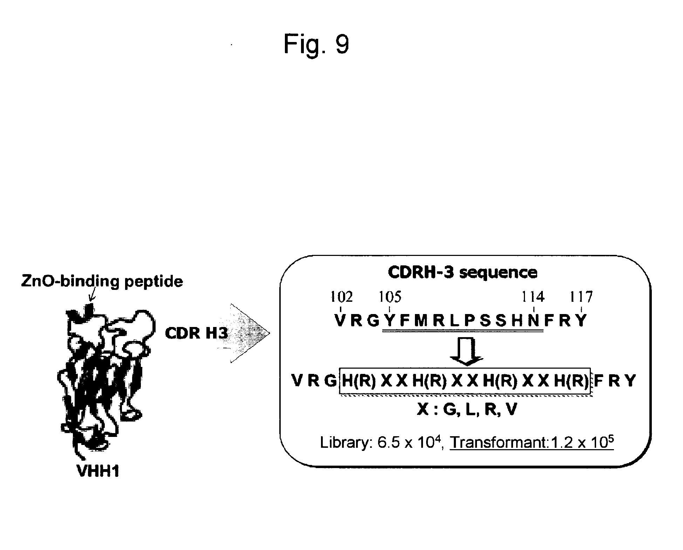

The library was constructed with a sequence shown in Fig. 9 ranging from residue 105 to residue 114 of the CDR-H3 region. Each gene corresponding to the H(R) site in Fig. 9 was designed so that the nucleotide sequence of DNA was "(C or A or G) (T or C or A)"; that is, the site will be His residue or Arg residue. Each gene corresponding to X was designed so that the nucleotide sequence was "(C or G) (T or G) (A or T or G or C)"; that is, X will be Gly residue or Leu residue or Arg residue or Val residue. The thus constructed gene library was introduced into a phagemid vector pTZ and presented on M13 phage, so that a VHH1 library was constructed as the antibody fragment library. Specifically, gene fragments obtained via randomization of CDR H-3 were amplified by overlap extension PCR using the peptide-grafted antibody VHH1 as a template and then introduced into a phagemid vector pTZ (Fig. 1B). The composition of a PCR solution and reaction conditions are similar to those listed in Table 1 of Example 1. Primer sequences for mutagenesis are as shown below.

### CDRH-3 randomization primers

VHH3-*Nco* I (Reverse) 5'-NNNCCATGGCCCAGGTTCAGCTGGTTGAAAG-3' (SEQ I D NO: 35) 
H3-random (Reverse) 5'-ATTGCGCAGCAGTGCGCGGTCRCSKTSKTCRCSKTSKTCRC
SKTSKTCRCTTTCGCTATTGGGGCCAGGG-3' (SEQ ID NO: 36)
 
H3-random (Forward) 5'-CCCTGGCCCCAATAGCGAAAGYGAMSAMSGYGAMSAMS
GYGAMSAMSGYGACCGCGCACTGCTGCGCAAT-3' (SEQ ID NO: 37)
 VHH3-sacII (Forward) 5'-NNNCCGCGGATGAAACGGTAACCTGGG-3' (SEQ ID NO: 38)

In a phagemid vector pTZ used in this study, a lac promoter is placed in a position opposite to that of a gene of interest and mRNA transcription is initiated from a phage-derived gVI promoter located at the g3p terminus. Therefore, production of a fusion protein with g3p was appropriately suppressed, enabling the display of a greater number of types of clones without causing gene deficiency. The *Escherichia coli* DH12S strain was transformed with the thus constructed phagemid vector by electroporation, and then subjected to successive culture in 20 ml of LB medium at 37°C, O/N, followed by plasmid extraction. The resultant was used as a phagemid library for procedures of a phage display method. The scale of the thus constructed library was 6.5 × 10⁴ and that of a transformant was 1.2 × 10⁵.

Next, zinc oxide-specific camel antibodies with higher affinity were selected from the thus constructed phage antibody library. It had been confirmed by a previous experiment that phosphoric acid binds to a zinc oxide surface, so as to prevent adsorption of proteins. A solution containing the phage antibody library was mixed with zinc oxide particles and then phages with antibody display incapable of binding to or capable only of weakly binding to zinc oxide were removed by (washing) using an aqueous solution containing phosphoric acid (10-50 mM) and a surfactant (0.05% Tween-20). Phages that had strongly bound were removed using an aqueous high-concentration phosphoric acid solution (200 mM). The thus removed phages were amplified again.

Specifically, 840 µl of the constructed phage antibody library was added to ZnO powder (0.2 mg), followed by 1 hr of mixing. Molecules not specifically adsorbed to ZnO were washed off using a 10 mM phosphate (pH 7.4)/200 mM NaCl/0.05% Tween20 solution and then a bound phage antibody was eluted using a 0.2 M phosphate buffer (pH 7.4, 200 mM NaCl). The *Escherichia coli* JM109 strain was cultured until O.D.₆₀₀ₙₘ = 0.5 was achieved, was infected again with the thus collected phage antibody for amplification, and then cells were seeded over plates. *Escherichia coli* containing phage genes on the plates was collected and then phage antibodies were prepared again, followed by repetition of panning. To obtain phage antibodies firmly binding to ZnO, the phosphate concentration for washing was increased in every round. The above procedures were repeated for 4 rounds, so that antibodies capable of binding to ZnO were concentrated. Table 3 shows various conditions.

**Table 3**

| | | |
|---|---|---|
| Antigen | | ZnO: 0.2 mg (specific surface area: 9.7 m²/mg and particle size: 100 nm (Hosokawa Powder Technology Research Institute)) |
| Conditions for washing | 1^{st} | 10 mM phosphate (pH 7.4)/200 mM NaCl/0.05% Tween 20 |
| | 2^{nd} | 30mM phosphate (pH 7.4)/200 mM NaCl/0.05% Tween 20 |
| | 3^{rd} | 50 mM phosphate (pH 7.4)/200 mM NaCl/0.05% Tween 20 |
| | 4^{th} | 50 mM phosphate (pH 7.4)/200 mM NaCl/0.05% Tween 20 |
| Conditions for elution | | 0.2 M phosphate (pH 7.4)/200 mM NaCl |

As a result, camel antibodies comprising CDR-H3 having the amino acid sequences shown in Table 4 were selected. The affinity of each camel antibody to zinc oxide was evaluated using a Langmuir plot. The results shown in Table 5 were obtained, so that 4F2 was confirmed to have the highest affinity.

**[Table 4] Selected clones**

| | |
|---|---|
| 3D2 | HLGHGGHRLH |
| 3E2 | HLGHGGHGLH |
| 4D4 | H GHGLHG R |
| 4F2 | HLGHGLHR H |

**[Table 5]**

| Saturated binding level and dissociation equilibrium constant (Kd) of each clone (Ws) | | |
|---|---|---|
| | **Ws [mol/m²]** | **K*_{d}* [M]** |
| **VHHl** | **1.52 × 10⁻⁷** | **1.76 × 10⁻⁷** |
| **4F2** | **1.40 × 10⁻⁷** | **9.39 × 10⁻⁹** |
| **4D4** | **1.48 × 10⁻⁷** | **2.55 × 10⁻⁸** |
| **3D2** | **1.67 × 10⁻⁷** | **9.84 × 10⁻⁸** |
| **3E2** | **1.19 × 10⁻⁷** | **2.10 × 10⁻⁸** |

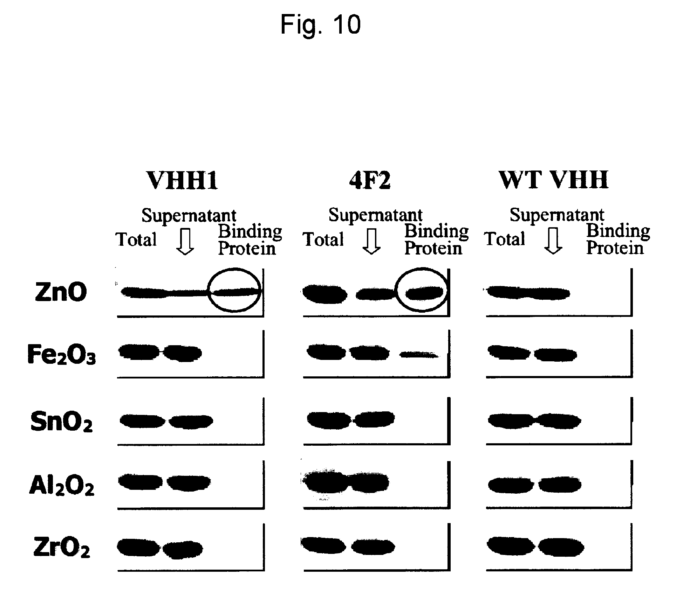

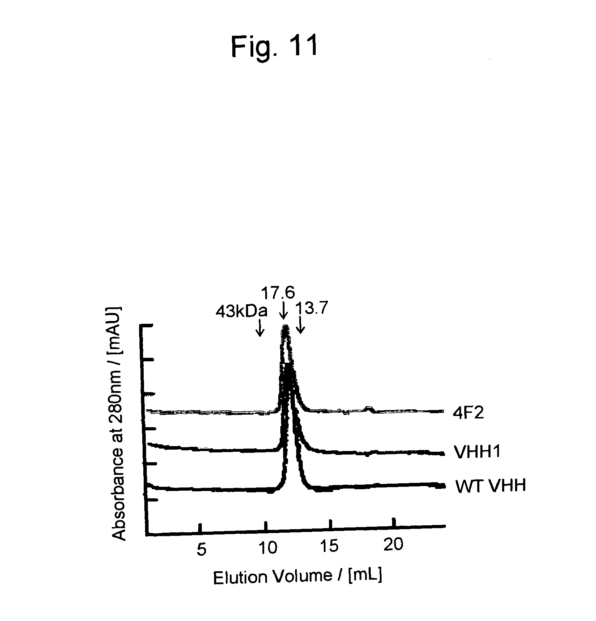

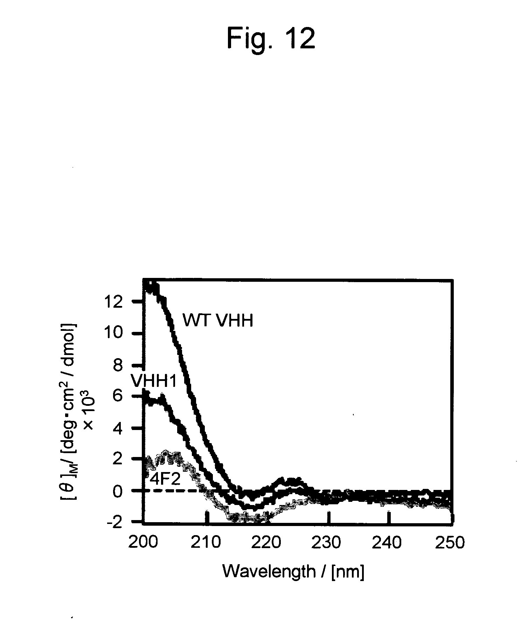

4F2 was also evaluated for material selectivity (Fig. 10). Specifically, the binding of 4F2 to iron oxide and titanium oxide was examined in a manner similar to that employed for zinc oxide. It was thus revealed that 4F2 bound to them to some extent, but had strong selectivity only for zinc oxide. Furthermore, the thus prepared 4F2 was evaluated by gel filtration chromatography and circular dichroism measurement. It was thus confirmed that 4F2 was a monomeric single component (orange line in Fig. 11), exerted the circular dichroism of β structure characteristic to antibodies (orange line in Fig. 12), and maintained an antibody molecular structure similar to that of a wild-type. Table 6 shows the full-length amino acid sequence (SEQ ID NO: 14) of 4F2.

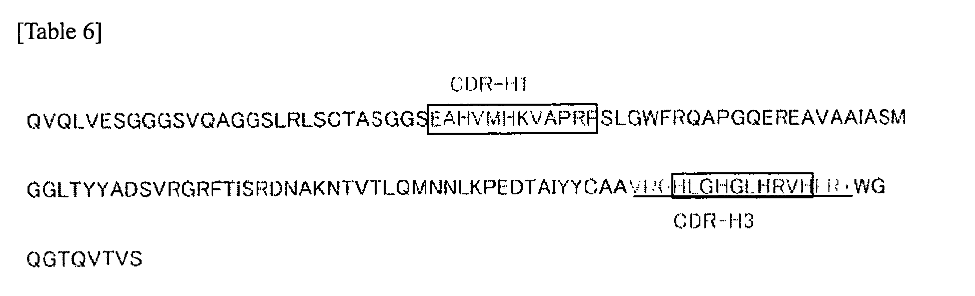

### Example 3: Improvement of dispersibility of zinc oxide particles using camel antibody 4F2

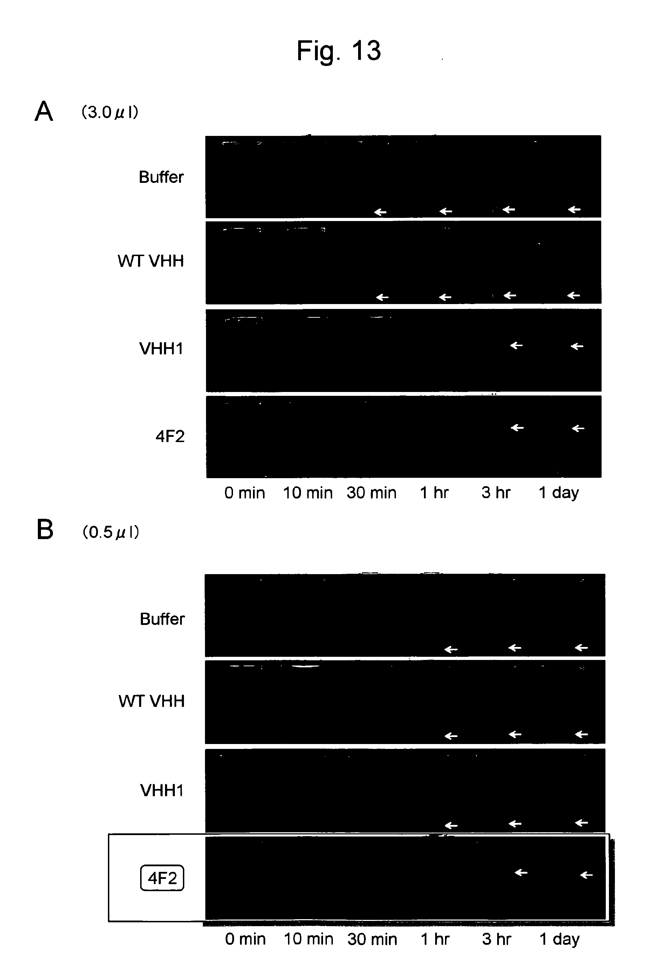

To 1 ml of an aqueous antibody fragment (WT VHH, or VHH1 or 4F2) solution (10 mM phosphate buffer), 0.5 mg of zinc oxide fine particles (particle size: 15-35 nm) was added. After 10 seconds of ultrasonication, dispersibility of particles was evaluated. When the concentration of the antibody fragment was 3 µM, zinc oxide particles retained dispersibility even after 1 day because of the use of VHH1 or 4F2 (Fig. 12). However, when the antibody concentration was 0.5 µM, dispersibility of fine zinc oxide particles was maintained only in the case of 4F2 (Fig. 13).

### Example 4: Dissociation of immobilized zinc oxide-binding antibody

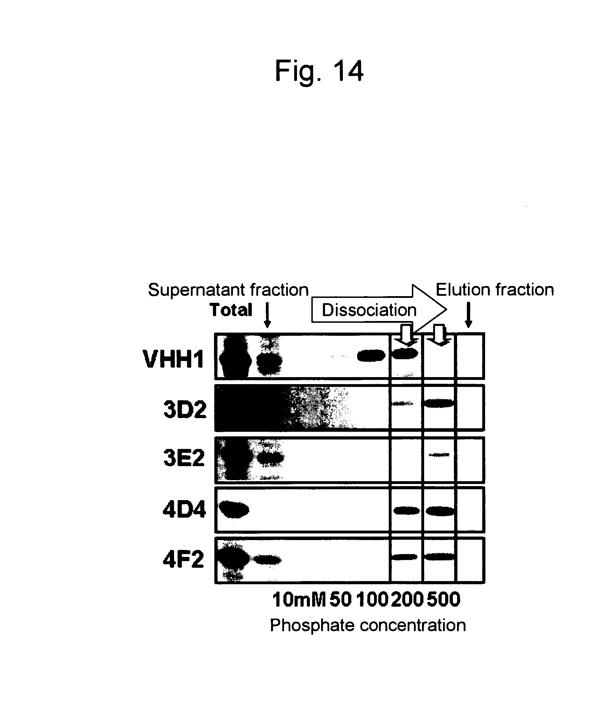

An aqueous zinc oxide-binding antibody solution was mixed with zinc oxide particles and then centrifuged. Then, zinc oxide particles were removed and phosphate buffers with varied phosphate concentrations were added to the particles. Dissociation of antibodies to supernatants was measured (Fig. 14). All antibodies could be dissociated from a zinc oxide surface by, after binding of such antibodies to zinc oxide, appropriately preparing a phosphate buffer having a phosphorus concentration of 10 mM or more and 500 mM or less.

### Example 5: Preparation of zinc oxide-gold linked protein prepared by fusing 4F2 to gold-recognizing antibody

The 4F2 antibody exerting high affinity for ZnO and Au VHH1 binding to Au were fused using a hinge linker, so that a bispecific antibody was prepared. With the use of overlap extension PCR, a previously reported Au-binding peptide-grafted antibody AuVHH1 and the ZnO-binding antibody 4F2 were bound via the following linker: Llama IgG2 upper hinge (EPKIPQPQPKPQPQPQPQPQPKPQPKPEP (SEQ ID NO: 39)), and 4F2-AuVHH1 gene was prepared. According to Example 1, an expression vector pRA4F2-AuVHH1 (Fig. 1C) in which the gene had been incoporated was constructed using the following primers.

### Primers for preparation of 4F2-AuVHH1

T7 promoter 5'-GAAATTAATACGACTCACTATAGGG-3' (SEQ ID NO: 40) VHH-Eag I-Hinge (Forward) 5'-TTGCGGTTGTGGTTGCGGTTGCGGTTTCGGCTGCG 
GCTGCGGAATTTTCGGTTCGGCCGATGAAACGGTAACCTGGGTG-3' (SEQ ID NO:
41)
 
Hinge-Bam-VHH (Reverse) 5'-CAACCGCAACCACAACCGCAACCGCAGCCGAAACC
GCAGCCGAAACCGGAACCGGATCCCAGGTTCAGCTGGTTGAAAGC-3' (SEQ ID
NO: 42)
 T7 terminater 5'-GCTAGTTATTGCTCAGCGG -3' (SEQ ID NO: 43)

The surfaces of gold materials and zinc oxide particles, or zinc oxide particles and gold nanoparticles can be linked via the fusion protein.

### Example 6: Evaluation of binding activity using surface plasmon resonance

### 1. Evaluation of zinc oxide-binding antibody for zinc oxide binding activity

A zinc oxide film was prepared on a gold film sensor chip for SPR measurement, Sensor Chip Au (BIAcore). Affinity of an antibody for the zinc oxide film was measured using BIAcore® 2000. Specifically, a zinc oxide film with a film thickness ranging from several nm to 50 nm was prepared by a sputtering technique or the like on a gold plate surface of Sensor Chip Au. Subsequently, the plate was placed within BIAcore® 2000, an aqueous antibody solution was loaded, and then adsorption of the antibody to the zinc oxide film was measured.

In the case of a zinc oxide film, the crystal structure can be altered by varying conditions for preparation. Hence, adsorption properties (the following association (binding) rate (ka), dissociation rate (kd), dissociation constant (KD), and saturated binding level) of an antibody due to differences in crystallinity of oxidized films were also evaluated. Quantitative evaluation can be carried out by calculating, based on a binding curve obtained according to analysis software attached to the above system, the binding rate (ka), dissociation rate (kd), and dissociation constant (KD) of the antibody to zinc oxide.

### 2. Evaluation of zinc oxide-binding antibody-immobilized zinc-bound substrate for antigen binding activity

A microchannel was provided on a sensor chip (substrate) having a zinc oxide film to which the zinc oxide-binding antibody had been adsorbed in the previous section. A sample containing an analyte (antigen) was loaded through the channel at a given rate and then interactions between the antibody and the antigen in the sample were monitored in real time using the surface plasmon phenomenon.

Such a sensor chip itself, to which the zinc oxide-binding antibody of the present invention had been adsorbed, can be used as a sensor chip for analysis of the kinetics of antigen-antibody interactions.

### Example 7: Adsorption properties of zinc oxide-binding antibody to zinc oxide as measured using reflectometry and application of zinc oxide-binding antibody to protein chip-flow system sensor

Reflectometry is a technique for measuring the amount of a protein adsorbed to the surface of a sample plate by reflecting a laser off of the sample plate and then measuring the reflected light. With the use of a reflectometry biosensor array system Fluid-RIfS (Fluidware Technologies Inc.) produced with a combination of the principle of reflectometry and a microfluidic chip, a zinc oxide thin film and a microchannel were designed on the silicon substrate and then an aqueous protein solution was loaded. Thus, the amount of the protein adsorbed onto the zinc oxide substrate was measured.

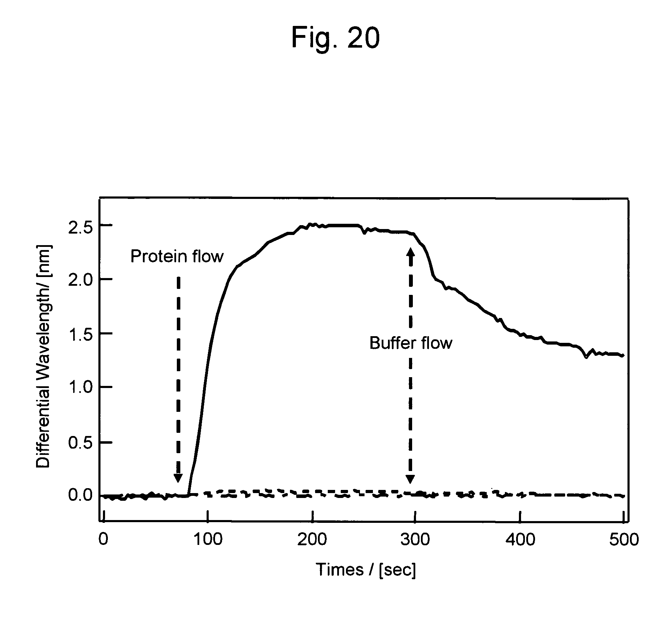

When the green fluorescent protein GFP was loaded, almost no adsorption of GFP onto the zinc oxide substrate was observed in 10 mM or 30 mM aqueous phosphoric acid solution (black dotted line and black broken line in Fig. 20). It was shown that almost no GFP had been physically adsorbed onto the zinc oxide surface. However, GFP prepared by fusing the zinc oxide-binding peptide (EAHVMHKVAPRP) to the N-terminus underwent clear adsorption to the zinc oxide substrate, such that the saturated binding level in 30 mM aqueous phosphoric acid solution was approximately 89 nmol/m² (Fig. 20). However, when a loading solution was exchanged with an aqueous protein-free phosphoric acid solution (dissociation step), peptide-fused GFP was partially dissociated.

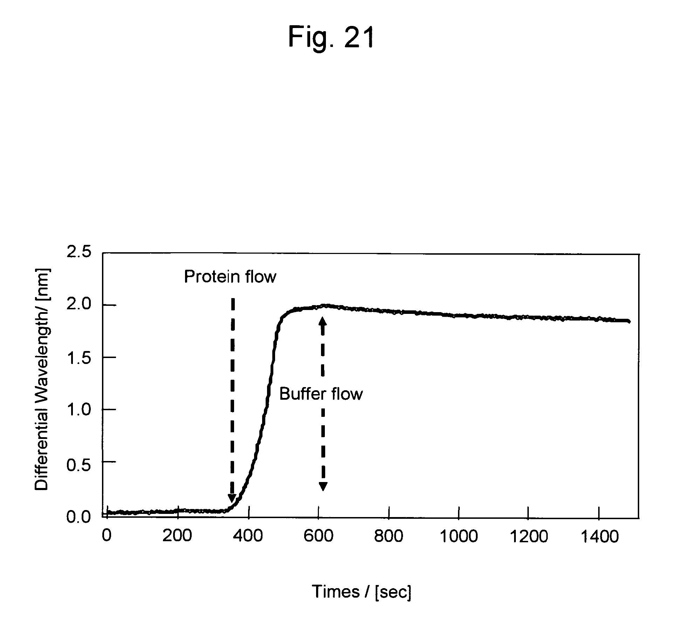

Next, a similar experiment was conducted using the zinc oxide-binding camel antibody 4F2. 4F2 underwent adsorption to a zinc oxide substrate in a manner similar to that of zinc oxide-binding peptide-fused GFP, but the amount of 4F2 adsorbed was approximately 130 nmol/m², which was approximately 1.5 times greater than that of the peptide. Also in the dissociation step, almost no protein dissociation was observed (Fig. 21). Based on this result, it could be confirmed that 4F2 having strong capability of binding to zinc oxide can be used as a tag that enables stable, strong, and rapid protein immobilization on a zinc oxide substrate.

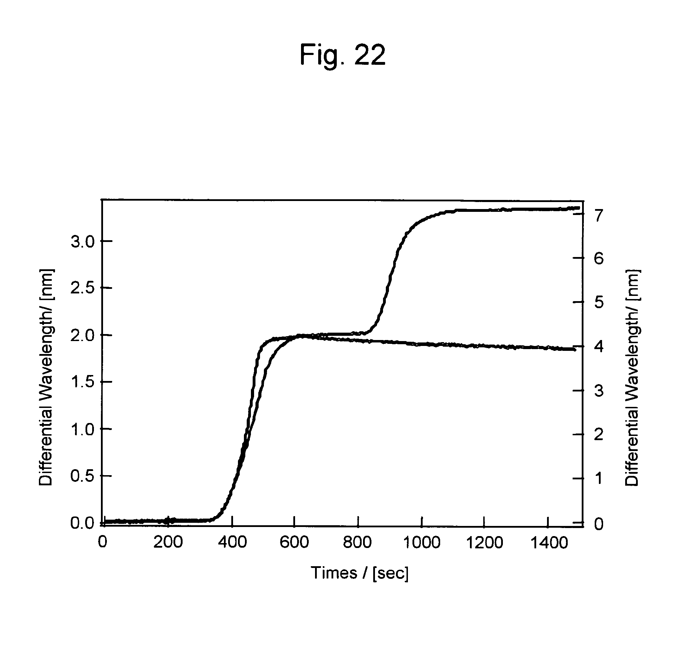

Moreover, 4F2 and a camel antibody (anti-GFP camel antibody) capable of binding to GFP were fused and then a biological reflective sensor for GFP detection was prepared and then evaluated. As a result, it could be confirmed that the anti-GFP camel antibody could be immobilized on a zinc oxide surface via 4F2 while maintaining the 80% of its activity and GFP could also be detected (upper line in Fig. 22).

### Comparative example 1: Preparation of zinc oxide-binding antibody using mouse-derived anti-hen egg white lysozyme antibody

According to Example 1, a zinc oxide-recognizing peptide was grafted into six (6) CDRs of an anti-hen egg white lysozyme antibody HyHEL-10 Fv (Protein Data Bank: 1C08, J. Biol. Chem. 274:27623-27631 (1999), three-dimensional structure: Fig. 16A) instead of a camel antibody.

Specifically, PCR primers were designed based on the sequence (EAHVMHKVAPRP) of a ZnO-binding peptide and then Overlap Extension PCR was carried out using an existing HyHEL-10 VH (or VL) gene-containing vector pRA-WT VH (or VL) in our laboratory as a template and the following primers, so that the peptide sequence was grafted into each CDR.

### Primers for construction of VH-2 (CDRH-2) and VH-3 (CDRH-3)

VH-*Nco* I 5'-NNNCCATGGCCGATATCCAGCTGCAG-3' (SEQ ID NO: 44)
VH-*Sac* II 5'-NNNCCGCGGAGACGGTGACGAGGGTGC-3' (SEQ ID NO: 45)
H2_Forward
5'-GCGCCACTTTATGCATCACATGCGCTTCGCCCATGTACTCGAGGCGGT-3' (SEQ ID NO: 46)
H2_Reverse
5'-TGTGATGCATAAAGTGGCGCCGCGTCCGCGCATCTCGATCACCCGCGA-3' (SEQ ID NO: 47)
H3_Forward
5'-GCGCCACTTTATGCATCACATGCGCTTCGTTCGCGCAGTAGTAGGTGG-3' (SEQ ID NO: 48)
H3_Reverse
5'-TGTGATGCATAAAGTGGCGCCGCGTCCGTGGGGCCAGGGCACCCTCGT-3' (SEQ ID NO: 49)

### Primers for construction of VL-2 (CDR L-2) and VL-3(CDR L-3)

Reverse primer
VL-*Nco* I 5'-NNNCCATGGCCGATATCGTCCTGACCC-3' (SEQ ID NO: 50)
VL-*Sac* II 5'-NNNCCGCGGCCTTGATCTCCAGCTTGG-3' (SEQ ID NO: 51)
L2_Forward
5'-GCGCCACTTTATGCATCACATGCGCTTCCTTGATCAGGAGGCGCGGG-3' (SEQ ID NO: 52)
L2_Reverse
5'-TGTGATGCATAAAGTGGCGCCGCGTCCGGGGATTCCGTCGCGCTTCAG-3' (SEQ ID NO: 53)
L3_Forward
5'-GCGCCACTTTATGCATCACATGCGCTTCGCAGAAGTACATGCCGAAGTC-3' (SEQ ID NO: 54)
L3_Reverse
5'-TGTGATGCATAAAGTGGCGCCGCGTCCGTTCGGCGGCGGCACCAAGC-3' (SEQ ID NO: 55)

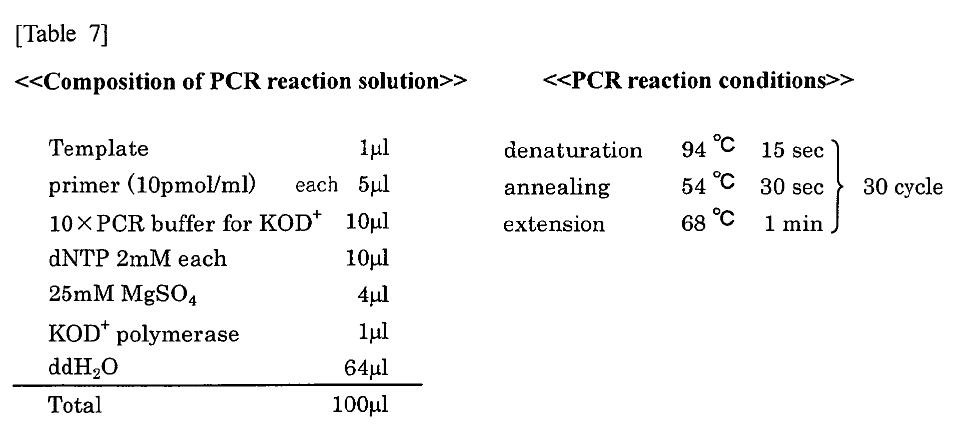

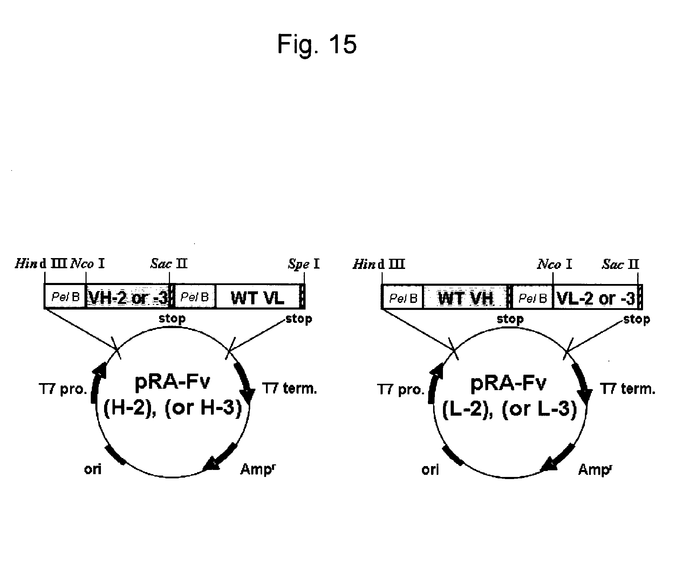

Next, each DNA fragment amplified by PCR and an existing expression vector pRA-scFv (HyHEL-10 scFv expression vector) in our laboratory were digested with restriction enzymes *Nco* I and *Sac* II and then linked, so that VH-2, VH-3, VL-2, and VL-3 expression vectors were constructed respectively. Furthermore, an existing HyHEL-10 Fv co-expression vectors pRA-pKTN2 VH and pRA-pKTN2 VL in our laboratory were digested with restriction enzymes *Nco* I and *Sac* II for re-ligation, so that Fv(H-2), (H-3), (L-2), and (L-3) co-expression vectors were constructed (Fig. 15).

Grafted antibodies were examined for ability to bind to zinc oxide. Peptide-grafted antibodies wherein the peptide had been grafted into heavy chain CDR-2 showed activity to bind to zinc oxide, but VH-VL dissociation phenomenon could not be avoided in the purification process (Fig. 16B). A peptide-grafted VH region alone was independently expressed in *Escherichia coli* and then purified, followed by circular dichroism measurement. Signals characteristic to antibodies were not shown, but signals indicating random structures were obtained (Fig. 16C).

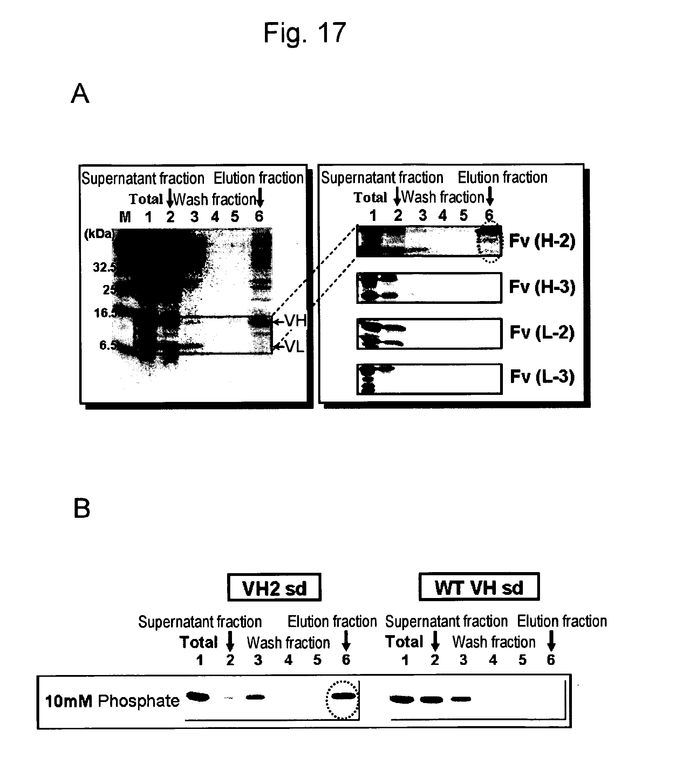

As described above, the mouse-derived anti-hen egg white lysozyme antibody was unable to avoid dissociation. Hence, binding to zinc oxide was evaluated directly using Fv secreted in a culture supernatant upon expression in *Escherichia coli* without purification of Fv (Fig. 17A). As a result, a peptide-grafted antibody wherein the peptide had been grafted into V chain CDR-H2 showed tha activity. So, this was purified in the form of a VH chain alone, and then its ability to bind to zinc oxide was evaluated (Fig. 17B). The VH chain showed the activity, but the structure was unstable. Thus, quantitative evaluation could not be carried out.

### Reference example 1: Evaluation of binding of short zinc oxide-recognizing peptide

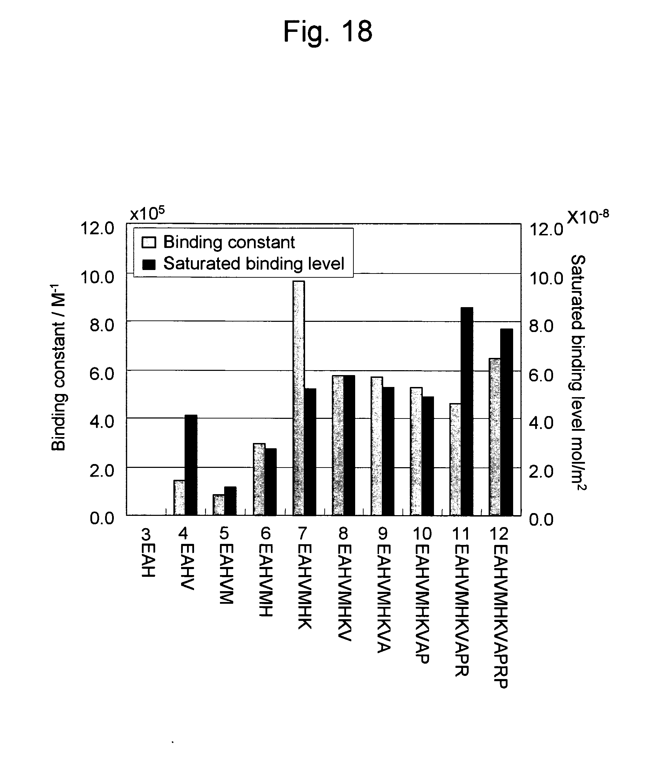

To examine a peptide length required for binding to zinc oxide, fragments of the zinc oxide-recognizing peptide (EAHVMHKVAPRP) were evaluated for binding (Fig. 18). As a result, the peptide (EAHVMHK) comprising 1-7 amino acid residues of the above sequence was found to exert the highest association equilibrium constant (reciprocal of dissociation equilibrium constant).

All publications, patents, and patent applications cited herein are incorporated herein by reference in their entirety.

### Industrial Applicability

According to the present invention, antibody molecules can be bound in a stable and specific orientation to zinc oxide. This technology can be used for development of high-throughput sensing technology such as biosensors and protein chips.

### Sequence Listing Free Text

SEQ ID NO: 1: zinc oxide-recognizing peptide (ZnO1)
SEQ ID NO: 2: zinc oxide-recognizing peptide (ZnO2)
SEQ ID NO: 3: zinc oxide-recognizing peptide (ZnO3)
SEQ ID NO: 4: zinc oxide-recognizing peptide (ZnO4)
SEQ ID NO: 5: zinc oxide-recognizing peptide (ZnO5)
SEQ ID NO: 6: sequence of zinc oxide-recognizing region
SEQ ID NO: 7: cAb BCII-10
SEQ ID NO: 8: zinc oxide-binding motif
SEQ ID NO: 9: sequence introduced into H-3 region
SEQ ID NO: 10: high-affinity mutant with sequence introduced into H-3 region (3D2)
SEQ ID NO: 11: high-affinity mutant with sequence introduced into H-3 region (3E2)
SEQ ID NO: 12: high-affinity mutant with sequence introduced into H-3 region (4D4)
SEQ ID NO: 13: high-affinity mutant with sequence introduced into H-3 region (4F2)
SEQ ID NO: 14: full-length amino acid sequence of 4F2
SEQ ID NO: 15: full-length amino acid sequence of VHH1
SEQ ID NO: 16: full-length amino acid sequence of VHH2
SEQ ID NO: 17: full-length amino acid sequence of VHH3
SEQ ID NO: 18: cAb BCII-10 CDR H-3 sequence (102-117 site) shown in Fig. 9
SEQ ID NO: 19: grafted sequence into cAb BCII-10 CDR H-3 shown in Fig. 9
SEQ ID NO: 20: gold-recognizing peptide
SEQ ID NOS: 21-38: primers
SEQ ID NO: 39: linker sequence (Llama IgG2 upper hinge)
SEQ ID NOS: 40-55: primers

## Claims

1. A zinc oxide-binding antibody, comprising:
a peptide-grafted antibody that contains a zinc oxide-recognizing peptide having an amino acid sequence comprising EAHVMHK in the CDR H-1 region of a camel antibody; or
a mutant that has a mutation in the CDR H-3 region of the peptide-grafted antibody and has an affinity for zinc oxide higher than that of the peptide-grafted antibody.

2. The zinc oxide-binding antibody according to claim 1, wherein the mutant contains a peptide comprising HXXHXXHXXH or HXXHXXHXXR (where X is G, L, R, or V) in the CDR H-3 region.

3. The zinc oxide-binding antibody according to claim 1, wherein the mutant contains a peptide comprising HLGHGGHRLH, HLGHGGHGLH, HVGHGLHGVR, or HLGHGLHRVH in the CDR H-3 region.

4. The zinc oxide-binding antibody according to any one of claims 1 to 3, which has the dissociation equilibrium constant K_{d} for zinc oxide lower than 1.7 × 10⁻⁷ [M].

5. The zinc oxide-binding antibody according to claim 4, wherein the dissociation equilibrium constant K_{d} for zinc oxide is lower than 9.5 × 10⁻⁹ [M].

6. The zinc oxide-binding antibody according to claim 5, wherein the zinc oxide-recognizing peptide is EAHVMHKVAPRP.

7. A zinc oxide-binding antibody, containing a zinc oxide-recognizing peptide comprising EAHVMHKVAPRP in the CDR H-1 region of a camel antibody and a peptide comprising HLGHGLHRVH in the CDR H-3 region.

8. The zinc oxide-binding antibody according to any one of claims 1 to 7, wherein the camel antibody has no disulfide bond between CDRs.

9. An expression vector comprising a gene encoding the zinc oxide-binding antibody according to any one of claims 1 to 8 and expressing a protein encoded by the gene.

10. The expression vector according to claim 9, comprising a gene encoding the amino acid sequence shown by SEQ ID NO: 14 and expressing a protein comprising the amino acid sequence.

11. A method for producing a zinc oxide-binding antibody, comprising culturing a host cell into which the expression vector according to claim 9 or 10 is introduced and then collecting a zinc oxide-binding antibody from a culture.

12. A solid support comprising a zinc oxide layer on which the zinc oxide-binding antibody according to any one of claims 1 to 8 is immobilized.

13. A biosensor having a solid support comprising a zinc oxide layer on which the zinc oxide-binding antibody according to any one of claims 1 to 8 is immobilized, and a means for detecting intermolecular interaction via the antibody.

14. A fusion protein comprising the zinc oxide-binding antibody according to any one of claims 1 to 8 fused to a second protein.

15. The fusion protein according to claim 14, wherein the second protein contains a gold-recognizing peptide having the sequence LKAHLPPSRLPS.
